(19) Europäisches Patentamt
European Patent Office
Office européen des brevets

(11) Publication number: **0 475 185 A1**

# (12) EUROPEAN PATENT APPLICATION

(21) Application number: **91114335.2**

(22) Date of filing: **27.08.91**

(51) Int. Cl.[5]: **C12N 15/31**, C12P 21/02, C12Q 1/68, G01N 33/541, C07K 13/00

(30) Priority: **27.08.90 JP 224945/90**

(43) Date of publication of application: **18.03.92 Bulletin 92/12**

(84) Designated Contracting States: **CH DE FR GB LI**

(71) Applicant: **NIPPON FLOUR MILLS CO., LTD.**
**27-5, Sendagaya 5-chome**
**Shibuya-ku Tokyo(JP)**

(72) Inventor: **Seto, Yasuhiro**
**5-16, Matsugae-cho**
**Sagamihara-shi, Kanagawa-ken(JP)**
Inventor: **Futo, Satoshi**
**2-214, Totatezama-Haitsu, 4-3011-6, Iriya**
**Zama-shi, Kanagawa-ken(JP)**
Inventor: **Mitsuse, Shizuo**
**4-25-8, Morinosato**
**Atsugi-shi, Kanagawa-ken(JP)**
Inventor: **Matsuo, Kanako**
**2-D, 1003, Tsurugamine honcho, Asahi-ku**
**Yokohama-shi, Kanagawa-ken(JP)**
Inventor: **Tsuna, Mika**
**1-403, 10, Chuo 1-chome**
**Ebina-shi, Kanagawa-ken(JP)**

(74) Representative: **Hansen, Bernd, Dr.rer.nat. et al**
**Hoffmann, Eitle & Partner Patentanwälte**
**Arabellastrasse 4 Postfach 81 04 20**
**W-8000 München 81(DE)**

## (54) DNA's encoding surface antigen of mycoplasma hyopneumoniae, DNA fragments for primer, recombinant antigenic peptides and diagnostic method of mycoplasmal pneumoniae of swine using same.

(57) A surface antigen gene which codes for a membrane protein having a molecular weight of 46 kd present in the membrane of Mycoplasma hyopneumoniae (M.hp) capable of specifically causing hybridization only with the M.hp DNA and suitable for diagnosing Mycoplasmal pneumoniae of swine (MPS), a DNA fragment for primer included in the gene and a method for diagnosing MPS in which the DNA or the fragment thereof is used as well as a method for detecting M.hp, in which these substances are used. A recombinant peptide capable of specifically causing antigen-antibody reaction with the anti-M.hp antibodies and suitable for diagnosing MPS and a method for diagnosing MPS in which the recombinant peptide is used.

EP 0 475 185 A1

## Field of the Invention

The present invention relates to a DNA coding for a surface antigen suitable for use in diagnosing Mycoplasmal pneumoniae of swine (hereinafter referred to as "MPS") attributable to Mycoplasma hyopneumoniae (hereinafter referred to as "M.hp"), a DNA fragment for primer included in the DNA sequence and a method for diagnosing MPS in which the DNA or the DNA fragment is used as well as a method for detecting MPS-causative pathogene, Mycoplasma hyopneumoniae, in which the DNA and fragments thereof are used.

The present invention also relates to a recombinant surface antigenic peptide suitable for use in diagnosing MPS and a method for diagnosing MPS using the antigenic peptide.

## Prior Art

Mycoplasmal pneumoniae of swine (MPS) is attributable to a microorganism called Mycoplasma hyopneumoniae. The swine suffering from this disease takes a chronic course of disease in nature and the mortality rate thereof is low. However, the rate of incidence thereof is very high and hence swine raising industry suffers a tremendous amount of economical loss. For instance, the feed efficiency is lowered.

In accordance with the investigation performed by Ministry of Agriculture, Forestry and Fisheries, the majority of the swine presently forwarded to the Japanese slaughterhouses have lung lesions attributable to this disease and the investigation makes it clear that this disease spreads all over the country.

Up to now, there have been proposed various serologic diagnostic methods as diagnostic method for detecting for MPS, but presently they have not yet been widely used in the field. This is because the conventional serologic methods suffer from the problem of specificity. More specifically, there have been separated many bacteria of genus Mycoplasma from the swine other than M.hp which is the causative pathogene for this disease and common antigens are present in these bacteria of genus Mycoplasma originated from the swine and the presence thereof is a major obstacle in the serologic diagnosis.

MORI et al. have developed a diagnostic method which comprises providing a monoclonal antibody for M.hp, purifying an antigen specific to M.hp and serologically diagnosing this disease by means of the enzyme-linked immunosorbent assay (ELISA method) (Yasuyuki MORI et al., Isr. J. Med. Sci., 23, p. 657). More specifically, MORI et al. have found out that among a variety of antigenic components present in the membrane of M.hp, membrane proteins having molecular weights of about 76 kd and 46 kd respectively serve as strong immunogens, that the infected swine can produce, from the early stage of this disease, antibodies to these antigens, that the antibodies to these antigens are produced over a long time period after the infection thereof and that these antigens are M.hp antigens having relatively high specificity and they suggest that these antigens may serve as good indicators in the MPS diagnosis. This method makes use of a procedure called double sandwich method and is characterized in that the specific antigens are purified from the cultured bacterial cells of M.hp by the use of a monoclonal antibody and the enzyme immunoassay is carried out using the resulting specific antigen.

Presently, the diagnostic method, in which a monoclonal antibody to a specific antigen is used, seems to be one having very great practical utility, but suffers from some problems explained below.

(a) This method uses a specific antigen extracted from the cultured bacterial cells of M.hp, but the cultivation of M.hp is very difficult and the yield and productivity of the M.hp are very low. Moreover, the preparation of the culture medium requires the use of very expensive agents such as serum and the titer and yield of the antigen may widely vary depending on the culture lots.

(b) The practice of the double sandwich method in which a monoclonal antibody is used requires the use of a considerably complicated system and, therefore, it is necessary to investigate various factors such as the handling properties and storability of a kit for practicing the diagnostic method in the field.

As an example of the diagnostic method free of these problems, Japanese Unexamined Patent Publication (hereinafter referred to as "J.P. KOKAI") No. Hei 2-167079 discloses a DNA sequence capable of specifically causing hybridization only with the M.hp DNA and useful in the diagnosis of MPS, an antigenic polypeptide which specifically undergoes an antigen-antibody reaction with the serum of M.hp-infected swine and a method for diagnosing MPS using the same. In this patent, about two thirds of the base sequence of the entire antigen gene which codes for the membrane protein having a molecular weight of 46 kd present in the foregoing cytoplasmic membrane of M.hp is established, this DNA having the base sequence is introduced into an expression vector to thus transform a proper host and a desired polypeptide is produced using the resulting transformant.

Incidentally, the separation and identification of a specific microorganism interested is in general performed by making use of specific properties peculiar to the microorganism.

In case of the M.hp, various problems arise, for instance, it grows slowly and the resulting colony cannot be observed with naked eyes. For this reason, the separation and identification of the M.hp requires substantial technical experiences. In particular, the proliferation of M.hp requires a special culture medium similar to tissue culture mediums unlike other bacteria belonging to the genus Mycoplasma since it can grow only in such a special culture medium and this makes the cultivation thereof more difficult.

SUMMARY OF THE INVENTION

An object of the present invention is to provide a surface antigen gene capable of specifically causing hybridization only with the M.hp DNA and suitable for diagnosing MPS, a DNA fragment for primer included in the gene and a method for diagnosing MPS in which the DNA or the fragment thereof is used as well as a method for detecting MPS-causative pathogene, Mycoplasma hyopneumoniae, in which these substances are used.

A further object of the present invention is to provide a recombinant peptide capable of specifically causing antigen-antibody reaction with the anti-M.hp antibodies and suitable for diagnosing MPS and a method for diagnosing MPS in which the recombinant peptide is used.

The inventors of this invention have succeeded in the determination of the base sequence of the entire antigen gene which codes for a membrane protein having a molecular weight of 46 kd present in the membrane of M.hp and thus have completed the present invention.

The present invention provides a DNA which codes for a surface antigen of Mycoplasma hyopneumoniae, which has an amino acid sequence (SEQ ID NO: 1) represented by the following formula (I):

Met Lys Lys Met Leu Arg Lys Lys Phe Leu Tyr Ser Ser Ala Ile Tyr
5 10 15
Ala Thr Ser Leu Ala Ser Ile Ile Ala Phe Val Ala Ala Gly Cys Gly
20 25 30
Gln Thr Glu Ser Gly Ser Thr Ser Asp Ser Lys Pro Gln Ala Glu Thr
35 40 45
Leu Lys His Lys Val Ser Asn Asp Ser Ile Arg Ile Ala Leu Thr Asp
50 55 60
Pro Asp Asn Pro Arg Trp Ile Ser Ala Gln Lys Asp Ile Ile Ser Tyr
65 70 75 80
Val Asp Glu Thr Glu Ala Ala Thr Ser Thr Ile Thr Lys Asn Gln Asp
85 90 95
Ala Gln Asn Asn Trp Leu Thr Gln Gln Ala Asn Leu Ser Pro Ala Pro
100 105 110
Lys Gly Phe Ile Ile Ala Pro Glu Asn Gly Ser Gly Val Gly Thr Ala
115 120 125
Val Asn Thr Ile Ala Asp Lys Gly Ile Pro Ile Val Ala Tyr Asp Arg
130 135 140
Leu Ile Thr Gly Ser Asp Lys Tyr Asp Trp Tyr Val Ser Phe Asp Asn
145 150 155 160
Glu Lys Val Gly Glu Leu Gln Gly Leu Ser Leu Ala Ala Gly Leu Leu
165 170 175
Gly Lys Glu Asp Gly Ala Phe Asp Ser Ile Asp Gln Met Asn Glu Tyr
180 185 190

Leu Lys Ser His Met Pro Gln Glu Thr Ile Ser Phe Tyr Thr Ile Ala

195 200 205

Gly Ser Gln Asp Asp Asn Asn Ser Gln Tyr Phe Tyr Asn Gly Ala Met

210 215 220

Lys Val Leu Lys Glu Leu Met Lys Asn Ser Gln Asn Lys Ile Ile Asp

225 230 235 240

Leu Ser Pro Glu Gly Glu Asn Ala Val Tyr Val Pro Gly Trp Asn Tyr

245 250 255

Gly Thr Ala Gly Gln Arg Ile Gln Ser Phe Leu Thr Ile Asn Lys Asp

260 265 270

Pro Ala Gly Gly Asn Lys Ile Lys Ala Val Gly Ser Lys Pro Ala Ser

275 280 285

Ile Phe Lys Gly Phe Leu Ala Pro Asn Asp Gly Met Ala Glu Gln Ala

290 295 300

Ile Thr Lys Leu Lys Leu Glu Gly Phe Asp Thr Gln Lys Ile Phe Val

305 310 315 320

Thr Gly Gln Asp Tyr Asn Asp Lys Ala Lys Thr Phe Ile Lys Asp Gly

325 330 335

Asp Gln Asn Met Thr Ile Tyr Lys Pro Asp Lys Val Leu Gly Lys Val

340 345 350

Ala Val Glu Val Leu Arg Val Leu Ile Ala Lys Lys Asn Lys Ala Ser

355 360 365

Arg Ser Glu Val Glu Asn Glu Leu Lys Ala Lys Leu Pro Asn Ile Ser

370 375 380

Phe Lys Tyr Asp Asn Gln Thr Tyr Lys Val Gln Gly Lys Asn Ile Asn

385 390 395 400

Thr Ile Leu Val Ser Pro Val Ile Val Thr Lys Ala Asn Val Asp Asn

405 410 415

Pro Asp Ala

According to one of preferred embodiments of the present invention, the DNA which codes for the surface antigen of Mycoplasma hyopneumoniae, which has the amino acid sequence represented by Formula (I) has a base sequence represented by the following formula (II)(SEQ ID NO: 2):

```
ATGAAAAAAA TGCTTAGAAA AAAATTCTTG TATTCATCAG CTATTTATGC AACTTCGCTT    60
GCATCAATTA TTGCATTTGT TGCAGCAGGT TGTGGACAGA CAGAATCAGG TTCGACTTCA   120
GATTCTAAAC CACAAGCCGA GACTCTAAAA CATAAAGTAA GTAATGATTC TATTCGAATA   180
GCACTAACCG ATCCGGATAA TCCTCGATGA ATTAGTGCCC AAAAAGATAT TATTTCTTAT   240
GTTGATGAAA CAGAGGCAGC AACTTCAACA ATTACAAAAA ACCAGGATGC ACAAAATAAC   300
TGACTCACTC AGCAAGCTAA TTTAAGTCCA GCGCCAAAAG GATTTATTAT TGCCCCTGAA   360
AATGGAAGTG GAGTTGGAAC TGCTGTTAAT ACAATTGCTG ATAAAGGAAT TCCGATTGTT   420
GCCTATGATC GACTAATTAC TGGATCTGAT AAATATGATT GGTATGTTTC TTTTGATAAT   480
GAAAAAGTTG GCGAATTACA AGGTCTTTCA CTTGCGGCGG GTCTATTAGG AAAAGAAGAT   540
GGTGCTTTTG ATTCAATTGA TCAAATGAAT GAATATCTAA AATCACATAT GCCCCAAGAG   600
ACAATTTCTT TTTATACAAT CGCGGGTTCC CAAGATGATA ATAATTCCCA ATATTTTTAT   660
AATGGTGCAA TGAAAGTACT TAAAGAATTA ATGAAAAATT CGCAAAATAA AATAATTGAT   720
TTATCTCCTG AAGGCGAAAA TGCTGTTTAT GTCCCAGGAT GAAATTATGG AACTGCCGGT   780
CAAAGAATCC AATCTTTTCT AACAATTAAC AAAGATCCAG CAGGTGGTAA TAAAATCAAA   840
GCTGTTGGTT CAAAACCAGC TTCTATTTTC AAAGGATTTC TTGCCCCAAA TGATGGAATG   900
GCCGAACAAG CAATCACCAA ATTAAAACTT GAAGGATTTG ATACCCAAAA AATCTTTGTA   960

ACTGGTCAAG ATTATAATGA TAAAGCCAAA ACTTTTATCA AAGACGGCGA TCAAAATATG  1020
ACAATTTATA AACCTGATAA AGTTTTAGGA AAAGTTGCTG TTGAAGTTCT TCGGGTTTTA  1080
ATTGCAAAGA AAAATAAAGC ATCTAGATCA GAAGTCGAAA ACGAACTAAA AGCAAAACTA  1140
CCAAATATTT CATTTAAATA TGATAATCAA ACATATAAAG TGCAAGGTAA AAATATTAAT  1200
ACAATTTTAG TAAGTCCAGT AATTGTTACA AAAGCTAATG TTGATAATCC TGATGCCTAA  1260
```

According to another aspect of the present invention, there is provided a method for diagnosing swine epidemic pneumonia which comprises detecting Mycoplasma hyopneumoniae by a DNA hybridization method using a DNA probe which comprises a DNA sequence represented by the foregoing formula (I) or (II) or a DNA probe which comprises at least part of the sequence comprising 1st to 414th nucleotides of the DNA sequence represented by Formula (II).

According to a further aspect of the present invention, there is provided a DNA fragment for primer which comprises a member selected from the group consisting of DNA sequences which comprise at least part of the sequence comprising 1st to 414th nucleotides of the DNA sequence represented by Formula (II) and complementary DNA sequences thereof, and a DNA fragment for primer which comprises a member selected from the group consisting of DNA sequences each of which is a part of the sequence which comprises 415th to 1260th nucleotides of the DNA sequence represented by Formula (II) and complementary DNA sequences thereof.

According to a further aspect of the present invention, there is provided a method for detecting Mycoplasma hyopneumoniae, which comprises the steps of adding two kinds of primers selected from the foregoing DNA fragments for primer to a solution to be examined, amplifying a target DNA by a polymerase-chain-reaction method (PCR method) and then detecting the amplified DNA fragment.

In a preferred embodiment of the present invention, the detection of the DNA fragment in the foregoing detection method is carried out by a DNA hybridization method using a DNA probe which comprises the whole or a part of the DNA sequence represented by Formula (II).

According to a further aspect of the present invention, there is provided a recombinant antigenic peptide which comprises the amino acid sequence represented by Formula (I) or a part thereof.

According to a further aspect of the present invention, there is provided a method for diagnosing Mycoplasmal pneumoniae of swine (MPS) wherein the above-mentioned recombinant antigenic peptide is brought into contact with a sample under the conditions that the peptide reacts with anti-M.hp antibodies present in the sample to form an immunological complex and the immunological complex is measured to confirm the existence of the antibodies in the sample.

BRIEF EXPLANATION OF THE DRAWINGS

Fig. 1 shows the results of M.hp detection through hybridization method by the use of the DNA of the present invention whose sequence corresponds to 1st to 414th bases of the sequence represented by Formula (II), as a probe;

Fig. 2 shows the results of M.hp detection through hybridization method by the use of the DNA sequence of the present invention which comprises a part of the sequence represented by Formula (II), as a probe;

Fig. 3 shows the results of M.hp detection obtained by the PCR method in which the primer DNA fragment of the present invention is used;

Fig. 4 shows the results of the detection of M.hp present in samples originated from infected swine obtained by PCR method in which the primer DNA fragment of the present invention is used; and

Fig. 5 shows the results of M.hp detection obtained by the combination of the PCR method and the DNA probe method in which the primer DNA fragment of the present invention is used.

DETAILED DESCRIPTIONS OF THE PREFERRED EMBODIMENTS

The present invention will further be described in detail below.

The PCR method used in the present invention is a technique developed in 1985 by Cetus Co., Ltd. and makes it possible to easily amplify a DNA having a known base sequence within a short period of time. According to this method, first there is prepared, synthetically or by other methods, complementary DNA's (called primer) of two kinds of short chain DNA's (for instance, those comprising 20 to 30 nucleotides) of a target double-stranded DNA, each comprising a 3'-terminal. To a sample to be treated, there are added these two primers, dATP, dGTP, dCTP and dTTP as well as a DNA polymerase. The double-stranded DNA molecule is dissociated into single-stranded DNA chains (step 1). Then the two kinds of the primers each is hybridized or annealed with each single-stranded DNA (step 2) and the resulting complementary DNA strands each is elongated from the 3'-teminal serving as an initiation point to prepare the double-stranded DNA (step 3). The double-stranded DNA thus synthesized is dissociated into single-stranded DNA's. One DNA molecule can be amplified to $10^5$ DNA molecules or more by repeating the foregoing steps 1 to 3, over 10 to 30 times.

The PCR method is detailed in the following articles:

Mullis, K.B., Methods in Enzymology, 1987, 155, pp. 335 - 350;
Saiki, R.K., Science, 1985, 230, pp. 1350 - 1354;
Saiki, R.K., Nature, 1986, 324, pp. 163 - 166; and
J.P. KOKAI No. Sho 61-274697.

The sensitivity of M.hp detection greatly varies depending on the part of the DNA sequence represented by Formula (II) selected as the target DNA to be amplified with the primers and, therefore, upon practicing the PCR method, the selection of the primers used is very important. These two kinds of the primers used in combination are selected so that they are, respectively, hybridized with the separate single-stranded DNA molecules originated from a single double-stranded DNA molecule. In addition, they are desirably selected and used in combination so that they are not superposed to one another and that the two 3'-termini thereof are hybridized with the separate single-stranded DNA's derived from one double-stranded DNA between two 5'-termini thereof. These primers must have a length sufficient for initiating the synthesis of the target DNA in the presence of polymerizing agents. The length of the primer in general ranges from 10 to 30 nucleotides and preferably 15 to 25 nucleotides. Specific examples of the primers of the present invention are as follows:

① 5' ATGAAAAAAA TGCTTAGAAA AAAAT 3' (SEQ ID NO: 3)
(1st to 25th bases of the DNA sequence of Formula (II))

② 5' GACAGA CAGAATCAGG TTCGACTTCA G 3' (SEQ ID NO: 4)
(95th to 121th bases of the DNA sequence of Formula (II))

③ 5' T TGCCCCTGAA AATGGAAGTG GAGT 3' (SEQ ID NO: 5)
(350th to 374th bases of the DNA sequence of Formula (II))

④ 5' A ACAAATGCAA TAATTGATGC AAGC 3' (SEQ ID NO: 6)
(complementary DNA corresponding to 81th to 57th bases)

⑤ 5' CTTTATG TTTTAGAGTC TCGGCTTG 3' (SEQ ID NO: 7)
(complementary DNA corresponding to 157th to 133th bases)

⑥ 5' CGGA ATTCCTTTAT CAGCAATTGT A 3' (SEQ ID NO: 8)
(complementary DNA corresponding to 414th to 390th bases)

⑦ 5' TGTT GCCTATGATC GACTAATTAC T 3' (SEQ ID NO: 9)
(417th to 441th bases of the DNA sequence of Formula (II))

⑧ 5' ACAATT ACTGGACTTA CTAAAATTG 3' (SEQ ID NO: 10)
(complementary DNA corresponding to 1226th to 1202th bases)

⑨ 5' TTAGGCATCA GGATTATCAA CATTAGC 3' (SEQ ID NO: 11)
(complementary DNA corresponding to 1260th to 1234th bases)

In the present invention, a combination of a primer selected from ①, ②, ③ and ⑦ with a primer selected from④ , ⑤ , ⑥ , ⑧ and ⑨ is, for instance, used to synthesize a target DNA having an arbitrary length by the PCR method. In this case, it is necessary to select these two kinds of primers so that the 3'-terminal of the former is situated upstream of the 5'-terminal of the latter and preferably upstream of the 3'-terminal thereof. If these two kinds of primers are selected in such a manner, the DNA sequence present in the domain existing between the 5'-termini of these two kinds of primers is replicated and amplified. In general, these two kinds of primers are preferably selected such that the length of the DNA sequence situated between the 5'-termini is at least 30 nucleotides. However, if the length is too long, the rate of amplification is reduced. Therefore, the length is preferably not more than 1,000 nucleotides.

The primers of the present invention can easily be synthesized by any appropriate method, for instance, using a commercially available DNA synthesizer. Examples of polymerizing agents are a variety of DNA polymerases such as Taq DNA polymerase (available from Boehringer Co., Ltd) and Ampli Taq DNA polymerase (available from Cetus Co., Ltd.).

The DNA fragments thus amplified by the PCR method are separated by electrophoresis using an agarose gel or an acrylamide gel or high performance liquid chromatography and they can easily be detected by staining with ethidium bromide or the like and then irradiating with ultraviolet rays. Moreover, the DNA fragments thus amplified by the PCR method can be certainly identified by a DNA hybridization method in which a DNA appropriately selected from DNA's coding for the surface antigen of M.hp is used as a probe after the aforementioned separation or without carrying out the separation.

As labelling substances for probes, there may be used, for instance, conventionally known radioisotopes, fluorescent compounds, enzymes and biotin.

The surface antigen gene of M.hp used in the present invention is a gene having high specificity which is present only in M.hp. Accordingly, the detection and identification of M. hp can be ensured by examining whether, or not, the antigen gene is present in DNA extracted from the microorganisms separated from tissues.

I. Cloning of the Gene Encoding a 46 kd Antigen Peptide of M.hp(46 kd antigen gene)

An outline of the method for cloning the gene encoding a 46 kd antigen peptide of M.hp(46 kd antigen gene) will be given below.

Mycoplasmal cells are collected from the culture of M.hp, lysed in a buffer containing SDS, followed by the extraction of DNA and purification thereof.

The DNA of M.hp is cleaved with a restriction enzyme HindIII, the resulting fragment is inserted into a plasmid pUC119 at the site cleaved with HindIII.

This plasmid is transformed into Escherichia coli as a host cell, colony hybridization is performed by the DNA probe method utilizing EcoRI fragments of pKUM1 and pKUM2 to select the bacterial cells containing a plasmid (pURR126) carrying the 46 kd antigenic gene. In this respect, E. coli JM 109 which comprises the plasmid pKUM1 or pKUM2 is deposited with Fermentation Research Institute (FRI) under the accession number of FERM P-10318 or P-10319 (see J.P. KOKAI No. Hei 2-167079).

The base sequence of the 46 kd antigen gene is determined by the dideoxy method.

The transcription-initiation point of the base sequence is determined by the primer-extension method.

Thus, the entire base sequence (1260 bp) of the 46 kd antigen gene is determined.

II. Detection of M.hp by the Use of DNA Probe

(1) Preparation of DNA Probe

The plasmid (pURR126) obtained by inserting a fragment containing the 46 kd antigen gene obtained through the cleavage with a restriction enzyme HindIII into the HindIII-cleaved site of the plasmid pUC119 is treated with HindIII to give a DNA sequence containing the entire base sequence of the 46 kd antigen gene. The DNA sequence may further be cleaved, with a proper restriction enzyme, into shorter DNA sequences. Further, a DNA having a proper length can be synthesized by the PCR method using the primer of the present invention and a DNA including at least part of the 46 kd antigen gene as a template chain. The DNA thus obtained is purified and labelled with digoxigenin using a DNA labelling kit (available from Boehringer Co., Ltd.) to give a DNA probe.

(2) Hybridization

The culture medium is filtered through a nylon filter (Hybond-N available from Amerscham Co., Ltd. or NYTRAN NY13N available from Schleicher & Schuell Co., Ltd.) and the filter is immersed in a solution containing 0.5 N NaOH and 1.5 M NaCl. Then the filter is immersed in a buffer: 0.5 M Tris-HCl-1.5 M NaCl (pH 7.5), dried and subjected to a prehybridization treatment (at 65°C for one hour) for inhibiting the occurrence of nonspecific reunion reaction. Then the DNA probe prepared in the procedure (1) is added and hybridization is carried out at 65°C overnight. Thereafter, the detection of the labelled DNA is performed in accordance with the guideline attached to the kit available from Boehringer Co., Ltd.

III. Detection of M.hp by PCR Method

The 46 kd antigen gene of M.hp corresponds to a specific genetic domain peculiar to M.hp. Therefore, the PCR method can be carried out using, as a primer, a DNA which is a part of the gene to amplify the specific genetic domain peculiar to M.hp or a part thereof. Thus, a very small amount of M.hp present in a sample can be detected. Bacteria other than M.hp do not have the foregoing genetic domain peculiar to M.hp and, therefore, are not amplified at all. Since the domain to which the DNA probe is bonded is amplified to an extent of not less than $10^5$ as compared with the conventional DNA probe methods, a sensitivity higher than that observed by the use of a radioactive probe can be accomplished even if a non-radioactive DNA probe is employed.

If the PCR method is used alone, there may sometimes be observed a high background in the analysis by gel electrophoresis. The fragment amplified by the DNA probe method can be identified after the completion of the PCR reaction to eliminate the influence of such a high background. This would make the detection of M.hp more reliable.

IV. Production of Recombinant Peptide

The production of the recombinant peptide of the present invention represented by the amino acid sequence of Formula (I) will be explained below. There are two methods for the expression of a recombinant peptide; one in which the peptide is expressed in the form of a fused protein using Escherichia coli and the other in which the peptide is directly expressed.

First, the method in which the peptide is expressed in the form of a fused protein using Escherichia coli is explained. M. hp recognizes the codon, TGA, which is recognized as a termination codon by ordinary living things, as a tryptophan codon. In order to express the antigenic peptide of the present invention in Escherichia coli, it is necessary to change base "A" at 210, 303 and 762 positions of Formula (II) to base "G". For this purpose, there may suitably be used conventional in vitro mutagenesis methods such as Kunkel method or recombinant PCR method. For example, the above-mentioned base substitution can be conducted using plasmid pURR126, in vitro mutagenesis kit (Mutan K: available from TAKARA SHUZO Co., Ltd.) and synthetic oligonucleotides in a conventional manner.

Only the antigen gene region of the thus modified pURR126 is isolated by, for example, PCR method and then introduced into StuI-SalI cleaved site of an Escherichia coli expression vector such as pMAL-c (Protein Fusion and Purification System, New England Biolab. Inc.) to prepare a recombinant plasmid which is introduced into Escherichia coli. The transformant thus prepared is cultured to express a fused protein. The transformant harboring the plasmid derived from pMAL-c produces a fused protein wherein an Escherichia coli maltose binding protein and the surface antigenic peptide are bound to each other. For example, Maltose binding protein (MBP) produced by the pMAL-c transformed strain binds to an amylose gel. This property can be used to purify the product. From the thus purified fused protein, the target antigenic peptide can be excised by, for example, Factor Xa treatment. After the Factor Xa treatment, the mixture is subjected to an amylose gel treatment to adsorb thereon free MBP and non-excised fused protein. Non-adsorbed fractions are collected and concentrated to obtain a purified sample. Purity of the purified sample can be determined by SDS electrophoresis or reactivity with anti-46 kd antigen monoclonal antibody.

Then, the method in which the recombinant antigenic peptide is directly expressed is explained. Only the antigen gene region of the modified pURR126 is isolated by PCR method and then introduced into SmaI cleaved site of an Escherichia coli expression vector pKK223-3 (available from Pharmacia Co., Ltd.) to prepare a recombinant plasmid. In this method, one can obtain a pure recombinant surface antigenic peptide which does not contain a protein region derived from Escherichia coli. A surface antigen gene containing a ribosome binding site can be isolated by conducting PCR reaction using appropriately selected 5'-terminal primer and 3'-terminal primer. The PCR reaction products thus obtained are digested with SmaI and introduced into an SmaI cleaved site of plasmid pKK223-3 (Pharmacia Co., Ltd.). The recombinant plasmid is introduced into Escherichia coli JM109. Base sequence analysis is conducted to isolate a clone containing a plasmid in which an initiation codon is located in a promoter region of the plasmid. The cloned transformant is cultured to produce the recombinant peptide.

For example, cultured bacterial cells are collected, washed, dispersed in an SDS-PAGE buffer, heated at 95 °C for 10 minutes and centrifuged. The supernatant is subjected to SDS-PAGE. Detection can be performed by immunoblotting using anti-surface antigenic monoclonal antibody or CBB staining.

The plasmid contained in the clone which produced the recombinant peptide was named pMHP46. The recombinant Escherichia coli harboring this plasmid produces the target antigenic pepetide in intracellular soluble and insoluble fractions and periplasmic fractions. The recombinant antigenic peptide in the periplasmic fractions has the same structure as that of natural surface antigenic peptide from which a signal sequence is eliminated.

V. Method for Diagnosing Mycoplasmal Pneumoniae of Swine

A method for diagnosing mycoplasmal pneumoniae of swine using the thus purified recombinant antigenic peptide, in particular a method according to ELISA method will be explained.

The purified recombinant antigenic peptide is appropriately diluted with a buffer and added to each well of a microplate. After allowed to stand at room temperature overnight, the wells are washed with Tris-buffered saline containing 0.05% Tween 20 (TBST) and blocked with TBST containing 1% skim milk. Serum of swine to be examined which is diluted with TBST containing 1% skim milk is added to each of the wells, allowed to stand at room temperature for two hours and washed with TBST. Peroxidase labelled anti-swine IgG serum which is diluted to an optimum concentration with TBST containing 1% skim milk is added to each of the wells, allowed to stand at room temperature for two hours and washed with TBST. As a substrate, 3, 3', 5, 5'-tetramethylbenzidine is added and reacted at room temperature for 15 minutes. SDS is added to stop the reaction. Absorbance of each of the wells is measured at 405 nm using a microplate reader. From the absorbance, one can confirm the existence of the antibodies present in the serum samples and judge the infection of mycoplasmal pneumoniae of swine.

Then, a method for detecting antibodies against M.hp according to ELISA method in which the recombinant antigenic peptide and the monoclonal antibody are used in combination, that is, a method for diagnosing mycoplasmal pneumoniae of swine will be explained.

The purified recombinant antigenic peptide can be used for the detection of M.hp using IgA monoclonal antibody disclosed in J.P.KOKAI No. Sho 62-273455. In the method of the patent application, an antigen extracted from the cell body of cultured M.hp with Tween 20 is bound to the IgA monoclonal antibody to detect antibodies against M.hp. In the present invention, it is possible to use the above-mentioned recombinant antigenic peptide instead of the Tween 20 extracted antigen. In this case, it is unnecessary to purify the recombinant antigenic peptide. For example, Escherichia coli which contains pMHP46 and produces the recombinant antigenic peptide is cultured according to the aforementioned method. The cell bodies are collected and suspended in TBST containing 0.02% sodium azide and 10 mM 2-mercaptoethanol. The suspension is subjected to sonication and ultracentrifugation. The supernatant is used as a crude antigen solution. According to the method disclosed in J.P.KOKAI No. Sho 62-273455, IgA monoclonal antibody 14-1 is diluted with 0.1M sodium carbonate buffer (pH 8.6) to an optimum concentration. The solution (0.1 ml) is added to each of 96 wells of a microplate. After allowed to stand at room temperature overnight, the wells are washed with Tris-buffered saline containing 0.05% Tween 20 (TBST). The crude antigen solution diluted to 1/500 or the M.hp Tween 20 extracted solution is added to each of the wells and reacted at room temperature for two hours. After washed with TBST, blocking is conducted by TBST containing 1% skim milk. Serum(0. 1 ml) of swine to be examined which is diluted with TBST containing 1% skim milk is added to each of the wells, allowed to stand at room temperature for two hours and washed with TBST. Peroxidase labelled anti-swine IgG serum (0.1 ml) which is diluted to an optimum concentration with TBST containing 1% skim milk is added to each of the wells, allowed to stand at room temperature for two hours and washed with TBST. As a substrate, 3, 3', 5, 5'-tetramethylbenzidine is added and reacted at room temperature for 15 minutes. SDS is added to stop the reaction. Absorbance of each of the wells is measured at 405 nm using a microplate reader. From the absorbance, one can confirm the existence of anti-M.hp antibodies present in the serum samples and judge the infection of mycoplasmal pneumoniae of swine.

EXAMPLES

The present invention will hereinafter be described in more detail with reference to the following Examples.

Reference Example 1: Extraction of M.hp-DNA and Determination of the Entire Base Sequence Thereof

M.hp ATCC 25934 (strain J) was obtained from Japanese Association of Veterinary Biologics.

The microorganism was proliferated in a BHL culture medium (Takashi YAMAMOTO (1982), Proc. 7th Int. Congr. Pig Vet. Soc. Mexico, 94).

The bacterial cells of M.hp were collected in the middle of the logarithmic growth phase at which the culture medium caused color change to orange-yellow color (pH 7.0).

The resulting culture was centrifuged at 7,000 rpm and 4 °C for 20 minutes and washed twice with 0.25 M NaCl solution. The bacterial cells thus obtained were used in the subsequent processes such as the DNA isolation.

The bacterial cells of M.hp (collected from 200 ml× 4 cultures) were suspended in 1.8 ml of 0.2M Tris-HCl (pH 9.0)-0.5M EDTA, 0.2 ml of 10% SDS solution was added to the suspension and the resulting mixture was maintained at 45 °C for 20 minutes. Then an equivalent volume of phenol saturated with TE buffer solution (10 mM Tris-HCl, 1 mM EDTA, pH 8.0) was added, gently stirred and allowed to stand at room temperature for 30 minutes. Further, an equivalent volume of chloroform-isoamyl alcohol (24 : 1) was added to the upper layer of the suspension, gently stirred and allowed to stand at room temperature for 20 minutes, followed by the addition of 3 volumes of cold ethanol (- 20 °C ) to the upper layer. The resulting DNA was recovered by winding it around a glass rod. The DNA thus recovered was washed with 70% ethanol solution and then air-dried. This was dissolved in 1 ml of TE buffer solution, 10μ g/ml of RNase A was added thereto to react these at 37 °C for one hour, followed by extraction with phenol and with chloroform-isoamyl alcohol and addition of ethanol to precipitate the reaction product. The product was washed with ethanol, air-dried, dissolved in 500 ml of TE buffer solution to give a preparation of DNA.

The resulting DNA of M.hp was digested with HindIII, inserted into a plasmid pUC119 at the site cleaved with HindIII, introduced into E. coli JM 109 strain to transform the same and inoculated on a plate of LB culture medium (containing ampicillin). After cultivating at 37 °C overnight, the resulting colonies were transferred onto a colony/plaque screen (available from NEN Co., Ltd.).

The colonies were treated with a 0.5 N NaOH-1.5 M NaCl solution (3 min. × 2 times), then with a 1M Tris-HCl (pH 7.0)-1.5 M NaCl buffer solution (3 min.× 2 times) and with 5× SSC (20 min.X one time) and then dried.

To inhibit any non-specific binding of the DNA, prehybridization was performed at 65 °C for one hour. Colony hybridization was carried out at 65 °C overnight by the use of the foregoing EcoRI-cleaved fragments of pKUM1 and pKUM2 as the DNA probes to give bacterial colonies containing a plasmid pURR126 which comprised the 46 kd antigen gene. The plasmid pURR126 was extracted from the colonies and the base sequence of the DNA which comprised the 46 kd antigen gene included in the plasmid was analyzed by kilo-sequencing (see Henikoff, S., Gene, 1984, 28, pp. 351-359; Yanisch-Perron, C., Vieira, J. and Messing, J., ibid, 1985, 28, pp. 103-119).

A DNA was synthesized from a complementary primer DNA at a proper position on an RNA while making use of the properties of a reverse transcriptase (having an ability of synthesizing DNA making use of an RNA as a template) and the length from the site of the primer to the 5'-terminal of mRNA was measured to determine the position of the 5'-terminal of mRNA (transcription-initiation point). The primer DNA was an oligonucleotide of 24-mer complementary to mRNA (complemental to the DNA sequence comprising from 81th to 58th bases of the sequence represented by Formula (II)) which was synthesized by a DNA synthesizer and the 5'-terminal thereof was labelled with $T_4$ polynucleotide kinase and a radioisotope ($\tau$ -$^{32}$P)ATP. The RNA used was extracted by an RNA extraction kit (available from Pharmacia Co., Ltd.) and the whole RNA fractions were used.

To a mixture of RNA (about 80 μ g), the labelled primer DNA (about 5× $10^5$ cpm) and 10 × hybridization buffer solution (4 μ l), there was added pure water so that the total volume was equal to 40 μ l. After treating at 65 °C for one hour and then at room temperature for 2 hours, 120 μ l of an extension buffer solution and 2 μ l (72 units) of a reverse transcriptase were added and the extension reaction was performed at 37 °C for one hour. Ethanol was added in an amount of 360μ l, the resulting mixture was allowed to stand at - 80 °C for 10 minutes and then centrifuged. To the precipitates formed, there was added 1 ml of a 70% ethanol solution followed by centrifugation to recover DNA/RNA. After drying the product, 5 μ l of a stop buffer solution and 1.2 μ l of TE were added and acrylamide gel electrophoresis was carried out to determine the length of the DNA and hence the transcription-initiation point.

The compositions of the buffer solutions used are as follows:

| o 10× Hybridization Buffer Solution | |
|---|---|
| Tris-HCl (pH 8.3) | 100 mM |
| EDTA | 10 mM |
| KCl | 2.5 mM |

| o Extension Buffer Solution | |
|---|---|
| Tris-HCl (pH 8.3) | 23 mM |
| KCl | 17 mM |
| $MgCl_2$ | 13 mM |
| DTT | 13 mM |
| dATP, dGTP, dCTP, dTTP | (each) 0.33 mM |
| actinomycin | 1 mg/ml |

o Stop Buffer Solution

95% formamide
20 mM EDTA
0.05% BPB
0.05% xylenecyanol FF

Example 1: Preparation of DNA Fragments Used in DNA Probe Method

(1) The plasmid pURR126 comprising the 46 kd antigen gene was treated with a restriction enzyme DdeI, the DdeI fragment of 295 nucleotides was purified by agarose gel electrophoresis and subjected to digoxigenin labelling by the use of a DNA labelling kit (available from Boehringer Co., Ltd.).
(2) In the amplification of a gene by the PCR method, it is necessary to use primers, respectively, complementary to the 3'-terminals of a double-stranded DNA. In this Example, two kinds of primers ① and ⑥ each comprising 25 nucleotides were synthesized by a DNA synthesizer (Model 391-EP available from ABI Company). A DNA comprising 1st to 414th bases of the sequence represented by Formula (II) was amplified by the PCR method utilizing the primers① and ⑥ thus obtained. As a template DNA, there was used a product obtained by inserting a HindIII fragment comprising the 46 kd antigen gene of M.hp into a plasmid pUC119.

| | |
|---|---|
| template DNA | 2.5 pmol |
| primer ① | 200 pmol |
| primer ⑥ | 200 pmol |
| 10× reaction buffer solution | 2.5μ l |
| 25 mM $MgCl_2$ | 2.5μ l |
| 2 mM dATP, dGTP, dCTP, dTTP | 2.5μ l |
| Taq DNA polymerase | 4 units |
| water to make | 25μ l |

The mixture was reacted in a program-incubator capable of programming the temperature conditions. The conditions therefor were as follows:

| | |
|---|---|
| pre-incubation | 94°C, 2 min. |
| step 1 | 94°C, 2 min. |
| step 2 | 50°C, 2 min. |
| step 3 | 72°C, 2 min. |

The cycle which comprised steps 1 to 3 was repeated 30 times.

| | |
|---|---|
| post-incubation | 72℃ , 5 min. |

The amplified DNA was extracted with chloroform/TE, then purified by agarose gel electrophoresis and label led with digoxigenin by means of a DNA labelling kit available from Boehringer Co., Ltd.

Example 2: Detection of M.hp by DNA Probe

A culture (100μ l) of M.hp (or M. hyorhinis) was filtered through a dot plate (for instance, DP-48 available from Advantech • Toyo Co., Ltd.) and the bacterial cells were adsorbed onto a nylon filter (NYTRAN NY13N available from Schleicher & Schuell Company).

After treating the adsorbed bacterial cells with 0.5 N NaOH-1.5 M NaCl (2 min.× 2 times), with 1M Tris-HCl (pH 7.0)-1. 5M NaCl (2 min.× 2 times) and with 20 × SSC (3 min. × one time) and then drying, pre-hybridization was performed at 65 °C for one hour, followed by addition of the DNA probes prepared in the procedure (1) or (2) in Example 1 and hybridization at 65 °C overnight. Then the labelled DNA was detected in accordance with the guideline attached to a kit available from Boehringer. The results observed when the DNA probe prepared in the procedure (2) of Example 1 are shown in Fig. 1. The same results were also obtained when the DNA probe prepared in the procedure (1) of Example 1 was used.

As seen from the foregoing, it was found that the DNA probes of the present invention can effectively be used for diagnosing MPS since they can specifically react with the chromosomal DNA of M.hp.

Example 3: Detection of M.hp by DNA Probe Method

In the same manner as in Example 1, primers ① , ③ , ④ , ⑤ and ⑥ (each comprising 25 nucleotides) were synthesized by a DNA synthesizer. In the same manner as in Example 1, DNA fragments having 81, 157 and 65 nucleotides respectively were obtained by the use of a combination of primers ① and ④ ; ① and ⑤ ; or ③ and ⑥ . The detection of M.hp was performed using the resulting DNA fragments as the probes in the same manner as in Example 2. The results obtained are shown in Fig. 2.

As seen from the foregoing, it was found that the DNA probes of the present invention could effectively be used for diagnosing MPS since they could specifically react with the chromosomal DNA of M.hp.

Example 4: Detection of M.hp by PCR Method

Fifty μ l each of cultures of M.hp, M. hyorhinis (M.hr) and M. flocculare (M.f) obtained after 3 days cultivation was centrifuged at 12,000 rpm for 5 minutes to collect bacterial cells. The resulting bacterial cells were treated with a buffer solution containing nonionic surfactants (Triton X-100, NP-40 and Tween 20) and Proteinase K and thereafter heat-treated at 95 °C for 10 minutes to give each sample. Moreover, the DNA extracted from M.hp and the plasmid pURR126 in which the HindIII fragment containing the 46 kd antigen gene was inserted were subjected to usual purification and then used in the PCR method.

M.hp was detected by the PCR method using combinations of primers (a) ① and ⑥ ; (b) ② and ⑨ ; and (c) ⑦ and ⑧ , all of which had been prepared in the same manner as in Example 2. The compositions of the reaction solution and the reaction conditions used are as follows:

Reaction Solution

| | |
|---|---|
| Sample DNA | 5 to 50 ng |
| (or bacterial cell lysate) | (25μ l) |
| primers | 10 pmol each |
| 10× reaction buffer solution | 5 μ l |
| 25 mM $MgCl_2$ | 5 μ l |
| 2 mM dATP, dGTP, dCTP, dTTP | 5 μ l |
| Taq DNA polymerase | 2.5 units |
| water to make | 50 μ l |

Two drops of a mineral oil were spreaded on the reaction solution.

The solution was reacted in a program-incubator capable of programming the temperature conditions. The conditions therefor were as follows:

| | |
|---|---|
| pre-incubation | 94 ° C , 4.5 min. |
| step 1 | 94 ° C , 1.4 min. |
| step 2 | 55 ° C , 2 min. |
| step 3 | 72 ° C , 2 min. |

The cycle which comprised steps 1 to 3 was repeated 30 times.

post-incubation 72℃ , 7 min.

After the completion of the reaction and the removal of the mineral oil, 5μ l of the reaction solution was subjected to agarose gel electrophoresis. The results thus obtained are shown in Fig. 3.

With regard to the M.hp DNA (Samples 4 and 5) and the plasmid including the 46 kd antigen gene (Samples 2 and 3), there were amplified a DNA fragment of 414 nucleotides for the combination (a) (primers ① and ⑥ ); a DNA fragment of 1166 nucleotides for the combination (b) (primers ② and ⑨ ); and a DNA fragment of 810 nucleotides for the combination (c) (primers ⑦ and ⑧ ), respectively.

The same DNA fragments were amplified for the M.hp bacterial cell lysate (Sample 7), but any amplification of DNA was not observed for M.hr and M.f bacterial cell lysates (Samples 8 and 9).

The results clearly indicate that M.hp can be detected in a high specificity and sensitivity by the amplification of the 46 kd antigen gene by the PCR method.

Example 5: Detection of M.hp Present in Clinical Sample of Infected Swine

A tracheo bronchial lavage and a homogenate of the lung obtained from swine infected with M.hp in the laboratory were subjected to the detection of M.hp by the PCR method.

(1) Preparation of Samples from Swine Infected with M.hp in the Laboratory

One month-old germfree swine was inoculated with $10^8$ M.hp bacterial cells through the nasal cavity. After the inoculation thereof, the test animal was bred for 4 weeks, then dissected to excise the lung. A tracheo bronchial lavage was obtained by injecting PBS through the respiratory tract and then recovering the solution, while a homogenate of the lung was obtained by emulsifying the lesions of the lung with PBS. Each Sample was prepared by centrifuging each solution at 2,000 rpm for 2 minutes to remove the inclusions such as lymphocytes.

(2) PCR Method

An M.hp bacterial cell lysate was prepared in the same manner as in Example 4 and the PCR method was carried out under the same conditions. The results obtained are shown in Fig. 4.

With regard to the tracheo bronchial lavage (Sample 2) and the lung homogenate (Sample 3), there were observed the amplification of a DNA fragment of 414 nucleotides for the combination (a) (primers ① and ⑥ ); a DNA fragment of 1166 nucleotides for the combination (b) (primers ② and ⑨ ); and a DNA fragment of 810 nucleotides for the combination (c) (primers ⑦ and ⑧ ) respectively.

These results indicate that, in accordance with the PCR method of the present invention, M.hp can easily be detected within a very short time period while directly using samples such as tracheo bronchial lavage and lung homogenate.

Example 6: Detection of M.hp by the Combination of PCR Method-DNA Probe Method

In the same manner as in Example 5, the PCR method was carried out using a tracheo bronchial lavage, M.hp bacterial cell lysate, M.hr bacterial cell lysate and M.f bacterial cell lysate, then the detection of these microorganisms was performed by a non-radioactive DNA probe method which comprised spotting each sample on a nylon filter while using a probe prepared by labelling the 46 kd antigen gene with digoxigenin using a kit available from Boehringer.

After the completion of the reaction by the PCR method, 5 $\mu$ l of the reaction solution was spotted onto a nylon filter, treated with an alkali, neutralized and then dried. The resulting filter was used in the subsequent hybridization.

As to the combination (a) (primers ① and ⑥ ), the fragment of a plasmid vector pURR130 (obtained by inserting a DNA sequence comprising 1st to 414th bases of the sequence represented by Formula (II) at the SmaI site of a plasmid pUC118) including a part of the 46 kd antigenic gene of M.hp obtained by cleaving the vector with restriction enzymes KpnI and BamHI was purified by agarose gel electrophoresis and was used as a DNA probe.

As to the combinations (b) (primers ② and ⑨ ); and (c) (primers ⑦ and ⑧ ), fragments of a plasmid vector pKUM1 including a part of the 46 kd antigen gene of M.hp obtained by cleaving the vector with a restriction enzyme EcoRI were purified by agarose gel electrophoresis and were used as DNA probes.

The DNA fragments were labelled with digoxigenin and the detection was performed by enzyme-labelled antibody technique. The results obtained are shown in Fig. 5.

This method had very high detection sensitivity as compared with the usual DNA probe methods and did not show any remarkable background.

Example 7: Modification of M.hp gene by in vitro mutagenesis

M.hp recognizes the codon, TGA, which is recognized as a termination codon by ordinary living things, as a tryptophan codon. In order to express the antigenic peptide of the present invention in Escherichia coli, base "A" at 210, 303 and 762 positions of Formula (II) were changed to base "G". The base substitution was conducted using plasmid pURR126, in vitro mutagenesis kit (Mutan K: available from TAKARA SHUZO Co., Ltd.) and synthetic oligonucleotides in a conventional manner.

Synthetic oligonucleotides used for the base substitution had 25 nucleotides and the following base sequences.

GGGCA CTAAT CCATC GAGGA T (SEQ ID NO: 12)

(complementary DNA corresponding to 220th to 200th bases of Formula (II), but base "T" at 210th position is replaced by base "C" undrelined)

GCTGA GTGAG CCAGT TATTT T (SEQ ID NO: 13)

(complementary DNA corresponding to 313th to 293th bases of Formula (II), but base "T" at 303th position is replaced by base "C" undrelined)

TTCCA TAATT CCATC CTGGG A (SEQ ID NO: 14)

(complementary DNA corresponding to 772th to 752th bases of Formula (II), but base "T" at 762th position is replaced by base "C" undrelined)

5'-Terminal ends of the above oligonucleotides prepared by a DNA synthesizer were phosphated as follows.

To the oligonucleotide (10 pmol), 5 × Kination buffer (2μ l) and T4 polynucleotide kinase (10 units), water was added to make 10 μ l. The mixture was treated at 37 °C for 15 minutes and then 70 °C for 10 minutes to prepare a phosphated synthetic oligonucleotide solution.

A single-stranded DNA having dU was prepared as follows.

Escherichia coli MV1184 was transformed with pURR126 containing HindIII fragments and the transformant was pre-cultured in LB medium (+Ap). The pre-cultured solution (30 μ l) was inoculated on 3 ml of 2× YT (+Ap) medium and was infected with pharge M13KO7 at moi (multiplicity of infection) of 2~ 10. The solution was allowed to stand at 37 °C for 30 minutes and kanamycin (70 μ g/ml) was added to kill cells not infected with the pharge. The solution was stirred at 37 °C overnight and centrifuged. The supernatant and 80 μ l of cultured solution of Escherichia coli BW313 (dut $^-$ ung $^-$ ) were mixed, allowed to stand at 37 °C for 10 minutes, inoculated on a plate of LB (Ap+) and cultured at 37 °C overnight. Single colonies were precultured in 2 × YT (+Ap) medium. The pre-cultured solution (1 ml) was inoculated on 100 ml of 2× YT medium and was infected with pharge M13KO7 at moi (multiplicity of infection) of 2 ~ 10. The solution was allowed to stand at 37 °C for 30 minutes and kanamycin (70 μ g/ml) was added to kill cells not infected with the pharge. The solution was stirred at 37 °C overnight and centrifuged. To the supernatant, 25 ml of NaCl/PEG was added and allowed to stand at room temperature for 10 minutes and then centrifuged. The precipitate was dissolved in 5 ml of TE (Tris-EDTA) buffer. Phenol/TE (5 ml) was added , stirred, allowed to stand at room temperature for 10 minmutes and centrifuged. To the aqueous layer, 5 ml of phenol/chloroform/TE was added, stirred, allowed to stand at room temperature for 10 minmutes and centrifuged. To the aqueous layer, 5 ml of c hloroform/isoamyl alcohol was added, stirred, allowed to stand at room temperature for 10 minmutes and centrifuged. To the aqueous layer, 500 μ l of $AcONH_4$ and 5 ml of isopropanol were added and centrifuged. The precipitate was washed with 70% alcohol solution, dried and dissolved in TE to prepare a single-stranded (ss) DNA solution.

Complementary chains were prepared as follows. The above ssDNA solution (0.2 pmol) and an annealing buffer (1 μ l) were dissolved in water to make 10 μ l solution. To one in l of the solution, one μ l of the phosphated synthetic oligonucleotide solution previously prepared was added and treated at 65 °C for 15 minutes and then at 37 °C for 15 minutes. To the solution, 25 μ l of the extension buffer solution, 60

units of E.coli DNA ligase and one unit of T4 DNA polymerase were added and allowed to stand at room temperature for two hours. Three μ l of 0.2 M EDTA (pH 8) was added and allowed to stand at 65 °C for 5 minutes to prepare a double-stranded (ds) DNA solution.

The dsDNA solution (3 μ l) was mixed with 30μ l of competent cell cultured solution of E.coli BMH 71-18 mut S (ung $^+$ ) and treated at 0°C for 30 minutes, 42 °C for 45 seconds and 0 °C for 3 minutes. The mixture was inoculated on 300 μ l of HCB (high competent broth: available from NIPPON GENE Co., Ltd.) and stirred at 37 °C for one hour. Ten μ l of M13KO7 was added and treated at 37°C for 30 minutes. One ml of 2 × YT (+Ap + kanamycin) was added, stirred at 37 °C overnight and centrifuged. The supernatant (20 μ l) and MV1184 cultured solution (80 μ l) were mixed, allowed to stand at 37 °C for 10 minmutes and centrifuged. Then, the solution was inoculated on a LB plate (+Ap) and cultured at 37°C overnight to prepare a transformant.

Example 8: Production of Recombinant Surface Antigenic Peptide by Fused Protein Expression Method and Diagnosis of Mycoplasmal Pneumoniae of Swine using the same

Only the antigen gene region of the modified pURR126 was isolated by PCR method and then introduced into StuI-SalI cleaved site of an Escherichia coli expression vector, pMAL-c (Protein Fusion and Purification System, New England Biolab. Inc.) to prepare a recombinant plasmid. The transformant harboring the plasmid derived from pMAL-c produces a fused protein wherein an Escherichia coli maltose binding protein and the surface antigenic peptide are bound to each other. The fused protein can be purified by amylose binding resin.

(1) Production and purification of recombinant antigenic peptide using pMAL-c

The surface antigen gene region of the modified pURR126 was isolated by PCR method. The primers used are as follows.

5'-terminal

GGAAG CCCGG GATGA AAAAA ATGCT TAGAA AAAAA TTCT (SEQ ID NO: 15)

(6th to 11th bases of this sequence, CCCGGG, is a linker for SmaI and 12th to 39th bases correspond to 1st to 28th bases of the sequence of Formula (II))

3'-terminal

GGAAG TCGAC TAACT TTTAG AAATT TTAGG CATCA GG (SEQ ID NO: 16)

(5th to 10th bases of this sequence, GTCGAC, is a linker for SalI and 11th to 37th bases are complementary DNA corresponding to 1275th to 1249th bases of the sequence of Formula (II) in which only 1st to 1260th bases are disclosed)

A PCR reaction using the above primers was conducted to isolate a surface antigen gene having additional one amino acid codon, GGG (for glycine) at 5'-terminal end. The PCR products were digested with SmaI-SalI and purified by agarose gel electrophoresis. The purified gene was introduced into StuI-SalI site of pMAL-c (available from NEB Co., Ltd.) and used to transform E.coli JM109.

Production of a fused protein by the strain thus prepared was conducted as follows.

200 μ l of overnight culture of the strain was inoculated on LB medium and cultured at 37 °C for 3 hours. An IPTG (isopropyl thiogalactoside) solution was added to make a final concentration of 0.3 mM and cultured for additional two hours.

The cells were collected, washed, suspended in SDS-PAGE buffer and heated at 95 °C for 10 minutes. After centrifugation, the supernatant was subjected to SDS-PAGE. The fused protein was detected by immuno-blotting using the anti-46 kd surface antigen monoclonal antibody and CBB staining. The plasmid contained in the strain having the highest productivity of the protein was named pMC46.

Finally, the strain which produced the recombinant protein in an amount of more than 10% by weight based on the total weight of the proteins produced by the E.coli strain.

The fused protein expressed by the strain containing pMC46 having introduced therein the M.hp surface antigen gene was purified according to the manual of NEB Inc. The maltose binding protein (MBP) produced by the pMAL-c transformed strain binds to an amylose gel. This property was used to purify the product. Further, the target antigenic peptide was excised from the thus purified fused protein by Factor Xa treatment.

Purifiaction of the fused protein

Intracellular soluble fractions of the strain containing pMC46 were subjected to amylose column chromatography.

Excision of the purified protein by Factor Xa

The fused protein purified by amylose gel column chromatography was excised by Factor Xa as follows. The protein was first dialysed against 8 M urea-TBS and then urea-TBS in which urea concentration was stepwise reduced to the final concentration of 0.5 M. This pretreatment increased the efficiency of excision.

Elimination of MBP

After the Factor Xa treatment, the protein was again subjected to amylose gel chromatography to adsorb thereon free MBP and non-excised fused protein. Non-adsorbed protein fractions were collected and concentrated to obtain a purified sample. Purity of the sample was determined by SDS electrophoresis and reactivity with the anti-46 kd antigen monoclonal antibody.

(2) Diagnosis of mycoplasmal pneumoniae of swine by ELISA

The recombinant antigenic peptide produced by the pMAL-c transformed strain was used to diagnose mycoplasmal pneumoniae of swine according to ELISA method.

The purified recombinant antigenic peptide was diluted with a 0.1 M $Na_2CO_3$ buffer (pH 9.0) to a concentration of 0.05 μ g/ml. The solution (0.1 ml) was added to each well of a 96 well microplate. After allowed to stand at room temperature overnight, the wells were washed with Tris-buffered saline containing 0.05% Tween 20 (TBST) and blocked with TBST containing 1% skim milk. Serum (0.1 ml) of swine to be examined which was diluted to 1/100 with TBST containing 1% skim milk was added to each of the wells, allowed to stand at room temperature for two hours and washed with TBST. Peroxidase labelled anti-swine IgG serum which was diluted to an optimum concentration with TBST containing 1% skim milk was added to each of the wells, allowed to stand at room temperature for two hours and washed with TBST. As a substrate, 3, 3', 5, 5'-tetramethylbenzidine was added and reacted at room temperature for 15 minutes. SDS was added to stop the reaction. Absorbance of each of the wells was measured at 405 nm using a microplate reader. The results are shown in Table 1.

Table 1 Detection of M.hp by ELISA method

| Swine | Sample | Absorbance at 405 nm | MPS |
|---|---|---|---|
| SPF | No.1 | 0.01 | - |
| | No.2 | 0.07 | - |
| | No.3 | 0.04 | - |
| | No.4 | 0.16 | - |
| | No.5 | 0.32 | - |
| M.hp infected | No.1 | 0.72 | + |
| | No.2 | 1.40 | + |
| | No.3 | 0.65 | + |
| | No.4 | 1.15 | + |
| | No.5 | 0.68 | + |
| | No.6 | 0.67 | + |
| M.f infected | No.1 | 0.38 | - |

SPF (specific pathogen free): Swine which were not infected with mycoplasmal pneumoniae and whose non-infection with M.hp was confirmed by bacteria separation method.

M.hp infected: Swine which were experimentally infected with M.hp and whose lung lesion was confirmed.

M.f infected: Swine which were experimentally infected with M. flocculare which is very allied to M.hp and very difficult to be serologically distinguished from M.hp.

MPS -: negative, +: positive

In this experiment, a sample whose absorbance at 405 nm is 0.5 or less is judged negative in terms of mycoplasmal pneumoniae.

Example 9: Production of Recombinant Surface Antigenic Peptide by Direct Expression Method and Diagnosis of Mycoplasmal Pneumoniae of Swine using the same

Only the antigen gene region of the modified pURR126 was isolated by PCR method and then introduced into SmaI cleaved site of an Escherichia coli expression vector, pKK223-3 (Pharmacia Co., Ltd) to prepare a recombinant plasmid. In this method, it is possible to obtain a pure recombinant surface antigenic protein which does not contain a protein derived from E.coli.

(1) Production and purification of recombinant antigenic peptide using pKK223-3

A PCR rection was conducted to isolate a surface antigen gene containing a ribosome binding site. The primers used were as follows.

5'-terminal

GGAAC CCGGG TTGTA TAATT GAATT AACTT GATTT GA (SEQ ID NO: 17)

(5th to 10th bases of this sequence, CCCGGG, is a linker for SmaI and 11th to 37th bases correspond to -38th to -12th bases of the sequence of Formula (II) in which only 1st to 1260th bases are disclosed)

3'-terminal

GGAAC CCGGG TAACT TTTAG AAATT TTAGG CATCA GG (SEQ ID NO: 18)

(5th to 10th bases of this sequence, CCCGGG, is a linker for SmaI and 11th to 37th bases are complementary DNA corresponding to 1275th to 1249th bases of the sequence of Formula (II) in which only 1st to 1260th bases are disclosed)

The PCR reaction products thus obtained were digested with SmaI and introduced into an SmaI cleaved site of plasmid pKK223-3 (Pharmacia Co., Ltd). The recombinant plasmid was introduced into Escherichia coli JM109. DNA sequence analysis was conducted to isolate a clone containing a plasmid in which an initiation codon was located in a promoter region of the plasmid. The cloned transformant was cultured to produce the recombinant peptide.

200 μ l of overnight culture of the strain was inoculated on 10 ml of LB medium and cultured at 37°C for 3 hours. To this solution, IPTG was added to a final concentration of 0.3 mM and cultivation was conducted for additional two hours.

The cells were collected, washed, dispersed in an SDS-PAGE buffer, heated at 95 °C for 10 minutes and centrifuged. The supernatant was subjected to SDS-PAGE. Detection was performed by immunoblotting using the anti-46 kd surface antigen monoclonal antibody and CBB staining.

The plasmid contained in the clone which produced the recombinant peptide was named pMHP46. The recombinant Escherichia coli harboring this plasmid produced the target antigenic peptide in intracellular soluble and insoluble fractions and periplasmic fractions. The recombinant antigenic peptide in the periplasmic fractions had the same structure as that of natural surface antigenic peptide from which a signal sequence was eliminated.

(2) Diagnosis of mycoplasmal pneumoniae of swine by ELISA using recombinant antigenic peptide and monoclonal antibody

The recombinant antigenic peptide can be used for the detection of M.hp using IgA monoclonal antibody disclosed in J.P. KOKAI No. Sho 62-273455. In the method of the patent application, an antigen extracted from the cell body of cultured M.hp with Tween 20 is bound to the IgA monoclonal antibody to detect anti-M.hp antibodies. In the present invention, it is possible to use the above-mentioned recombinant antigenic peptide instead of the Tween 20 extracted antigen. In this case, it is unnecessary to purify the recombinant antigenic peptide.

Escherichia coli which contains pMHP46 and produces the recombinant antigenic peptide was cultured according to the aforementioned method. The cells were collected and suspended in TBST containing 0.02% sodium aside and 10 mM 2-mercaptoethanol. The suspension was subjected to sonication and ultracentrifugation at 100,000 rpm for 30 minutes. The supernatant was used as a crude antigen solution.

According to the method disclosed in J.P.KOKAI No. Sho 62-273455, IgA monoclonal antibody 14-1 was diluted with 0.1M sodium carbonate buffer (pH 8.6) to an optimum concentration. The solution (0.1 ml) was added to each of 96 wells of a microplate. After allowed to stand at room temperature overnight, the wells were washed with Tris-buffered saline containing 0.05% Tween 20 (TBST). The crude antigen solution diluted to 1/500 or the M.hp Tween 20 extracted solution was added to each of the wells and reacted at room temperature for two hours. After washed with TBST, blocking was conducted by TBST containing 1% skim milk. Serum (0.1 ml) of swine to be examined which was diluted to 1/100 with TBST containing 1% skim milk was added to each of the wells, allowed to stand at room temperature for two hours and washed with TBST. Peroxidase labelled anti-swine IgG serum (0.1 ml) which was diluted to an optimum concentration with TBST containing 1% skim milk was added to each of the wells, allowed to stand at room temperature for two hours and washed with TBST. As a substrate, 3, 3', 5, 5'-t etramethylbenzidine was added and reacted at room temperature for 15 minutes. SDS was added to stop the reaction. Absorbance of each of the wells was measured at 405 nm using a microplate reader.

Table 2 Detection of M.hp by double sandwich method

| Swine Sample | | Absorbance at 405 nm | | | |
|---|---|---|---|---|---|
| | | Extracted | MPS | Recombinant | MPS |
| Control | | 0.000 | - | 0.000 | - |
| SPF | No.6 | 0.008 | - | 0.006 | - |
| | No.7 | 0.012 | - | 0.045 | - |
| | No.8 | 0.005 | - | 0.014 | - |
| | No.9 | 0.012 | - | 0.067 | - |
| | No.10 | 0.002 | - | 0.061 | - |
| M.hp infected | No.7 | 0.677 | + | 0.891 | + |
| | No.8 | 0.777 | + | 1.128 | + |
| | No.9 | 0.514 | + | 1.041 | + |
| | No.10 | 0.787 | + | 1.052 | + |
| | No.11 | 0.092 | - | 0.720 | + |
| | No.12 | 0.515 | + | 1.172 | + |
| M.f infected | No.2 | 0.041 | - | 0.081 | - |

SPF (specific pathogen free): Swine which were not infected with mycoplasmal pneumoniae and whose non-infection with M.hp was confirmed by bacteria separation method.

M.hp infected: Swine which were experimentally infected with M.hp and whose lung lesion was confirmed.

M.f infected: Swine which were experimentally infected with M. flocculare which is very allied to M.hp and very difficult to be serologically distinguished from M.hp.

MPS -: negative, +: positive

In this experiment, a sample whose absorbance at 405 nm is 0.5 or less is judged negative in terms of mycoplasmal pneumoniae.

The DNA of the present invention makes it possible to detect the chromosomal DNA peculiar to M.hp if it is used as a probe and, therefore, to easily and certainly detect M.hp and hence swine infected with M.hp.

Moreover, if the PCR method is carried out while using the DNA of the present invention as a primer, a very small amount of M.hp present in a sample can likewise be detected.

The recombinant antigenic peptide of the present invention can be produced in high yield by E.coli which can be cultured in low cost medium and has high specificity to anti-M.hp antibodies, stable antigenic property and no lot-to-lot variations.

The method of the present invention makes it possible to detect M.hp infection in its early stage, to perform intensive care therapy for preventing the outbreak of the swine epidemic pneumonia and thus to substantially reduce economical loss in the swine raising industry.

SEQUENCE LISTING

SEQ ID NO: 1

SEQUENCE TYPE: amino acid

SEQUENCE LENGTH: 419

TOPOLOGY: linear

MOLECULE TYPE: peptide

Met Lys Lys Met Leu Arg Lys Lys Phe Leu Tyr Ser Ser Ala Ile Tyr
5 10 15

Ala Thr Ser Leu Ala Ser Ile Ile Ala Phe Val Ala Ala Gly Cys Gly
20 25 30

Gln Thr Glu Ser Gly Ser Thr Ser Asp Ser Lys Pro Gln Ala Glu Thr
35 40 45

Leu Lys His Lys Val Ser Asn Asp Ser Ile Arg Ile Ala Leu Thr Asp
50 55 60

Pro Asp Asn Pro Arg Trp Ile Ser Ala Gln Lys Asp Ile Ile Ser Tyr
65 70 75 80

Val Asp Glu Thr Glu Ala Ala Thr Ser Thr Ile Thr Lys Asn Gln Asp
85 90 95

Ala Gln Asn Asn Trp Leu Thr Gln Gln Ala Asn Leu Ser Pro Ala Pro
100 105 110

Lys Gly Phe Ile Ile Ala Pro Glu Asn Gly Ser Gly Val Gly Thr Ala
115 120 125

Val Asn Thr Ile Ala Asp Lys Gly Ile Pro Ile Val Ala Tyr Asp Arg
130 135 140

Leu Ile Thr Gly Ser Asp Lys Tyr Asp Trp Tyr Val Ser Phe Asp Asn
145 150 155 160

Glu Lys Val Gly Glu Leu Gln Gly Leu Ser Leu Ala Ala Gly Leu Leu
165 170 175

Gly Lys Glu Asp Gly Ala Phe Asp Ser Ile Asp Gln Met Asn Glu Tyr
180 185 190

Leu Lys Ser His Met Pro Gln Glu Thr Ile Ser Phe Tyr Thr Ile Ala
195 200 205

Gly Ser Gln Asp Asp Asn Asn Ser Gln Tyr Phe Tyr Asn Gly Ala Met
210 215 220

Lys Val Leu Lys Glu Leu Met Lys Asn Ser Gln Asn Lys Ile Ile Asp
225 230 235 240

Leu Ser Pro Glu Gly Glu Asn Ala Val Tyr Val Pro Gly Trp Asn Tyr
245 250 255

Gly Thr Ala Gly Gln Arg Ile Gln Ser Phe Leu Thr Ile Asn Lys Asp
260 265 270

Pro Ala Gly Gly Asn Lys Ile Lys Ala Val Gly Ser Lys Pro Ala Ser
275 280 285

Ile Phe Lys Gly Phe Leu Ala Pro Asn Asp Gly Met Ala Glu Gln Ala
290 295 300

Ile Thr Lys Leu Lys Leu Glu Gly Phe Asp Thr Gln Lys Ile Phe Val
305 310 315 320

Thr Gly Gln Asp Tyr Asn Asp Lys Ala Lys Thr Phe Ile Lys Asp Gly
325 330 335

Asp Gln Asn Met Thr Ile Tyr Lys Pro Asp Lys Val Leu Gly Lys Val
340 345 350

Ala Val Glu Val Leu Arg Val Leu Ile Ala Lys Lys Asn Lys Ala Ser
355 360 365
Arg Ser Glu Val Glu Asn Glu Leu Lys Ala Lys Leu Pro Asn Ile Ser
370 375 380
Phe Lys Tyr Asp Asn Gln Thr Tyr Lys Val Gln Gly Lys Asn Ile Asn
385 390 395 400
Thr Ile Leu Val Ser Pro Val Ile Val Thr Lys Ala Asn Val Asp Asn
405 410 415
Pro Asp Ala

SEQ ID NO: 2

SEQUENCE TYPE: nucleotide

SEQUENCE LENGTH: 1260 base pairs

STRANDEDNESS: single

TOPOLOGY: linear

MOLECULE TYPE: genomic DNA

ORIGINAL SOURCE:

ORGANISM: Mycoplasma hyopneumoniae

NAME OF STRAIN: J

ATGAAAAAAA TGCTTAGAAA AAAATTCTTG TATTCATCAG CTATTTATGC AACTTCGCTT 60
GCATCAATTA TTGCATTTGT TGCAGCAGGT TGTGGACAGA CAGAATCAGG TTCGACTTCA 120
GATTCTAAAC CACAAGCCGA GACTCTAAAA CATAAAGTAA GTAATGATTC TATTCGAATA 180
GCACTAACCG ATCCGGATAA TCCTCGATGA ATTAGTGCCC AAAAAGATAT TATTTCTTAT 240
GTTGATGAAA CAGAGGCAGC AACTTCAACA ATTACAAAAA ACCAGGATGC ACAAAATAAC 300

```
TGACTCACTC AGCAAGCTAA TTTAAGTCCA GCGCCAAAAG GATTTATTAT TGCCCCTGAA   360
AATGGAAGTG GAGTTGGAAC TGCTGTTAAT ACAATTGCTG ATAAAGGAAT TCCGATTGTT   420
GCCTATGATC GACTAATTAC TGGATCTGAT AAATATGATT GGTATGTTTC TTTTGATAAT   480
GAAAAAGTTG GCGAATTACA AGGTCTTTCA CTTGCGGCGG GTCTATTAGG AAAAGAAGAT   540
GGTGCTTTTG ATTCAATTGA TCAAATGAAT GAATATCTAA AATCACATAT GCCCCAAGAG   600
ACAATTTCTT TTTATACAAT CGCGGGTTCC CAAGATGATA ATAATTCCCA ATATTTTTAT   660
AATGGTGCAA TGAAAGTACT TAAAGAATTA ATGAAAAATT CGCAAAATAA AATAATTGAT   720
TTATCTCCTG AAGGCGAAAA TGCTGTTTAT GTCCCAGGAT GAAATTATGG AACTGCCGGT   780
CAAAGAATCC AATCTTTTCT AACAATTAAC AAAGATCCAG CAGGTGGTAA TAAAATCAAA   840
GCTGTTGGTT CAAAACCAGC TTCTATTTTC AAAGGATTTC TTGCCCCAAA TGATGGAATG   900
GCCGAACAAG CAATCACCAA ATTAAAACTT GAAGGATTTG ATACCCAAAA AATCTTTGTA   960
ACTGGTCAAG ATTATAATGA TAAAGCCAAA ACTTTTATCA AAGACGGCGA TCAAAATATG  1020
ACAATTTATA AACCTGATAA AGTTTTAGGA AAAGTTGCTG TTGAAGTTCT TCGGGTTTTA  1080
ATTGCAAAGA AAAATAAAGC ATCTAGATCA GAAGTCGAAA ACGAACTAAA AGCAAAACTA  1140
CCAAATATTT CATTTAAATA TGATAATCAA ACATATAAAG TGCAAGGTAA AAATATTAAT  1200
ACAATTTTAG TAAGTCCAGT AATTGTTACA AAAGCTAATG TTGATAATCC TGATGCCTAA  1260
```

SEQ ID NO: 3

SEQUENCE TYPE: nucleotide

SEQUENCE LENGTH: 25 base pairs

STRANDEDNESS: single

TOPOLOGY: linear

MOLECULE TYPE: other nucleic acid (synthetic DNA for PCR)

```
ATGAAAAAAA TGCTTAGAAA AAAAT                                          25
```

SEQ ID NO: 4

SEQUENCE TYPE: nucleotide

SEQUENCE LENGTH: 27 base pairs

STRANDEDNESS: single

TOPOLOGY: linear

MOLECULE TYPE: other nucleic acid (synthetic DNA for PCR)

GACAGACAGA ATCAGGTTCG ACTTCAG 27

SEQ ID NO: 5

SEQUENCE TYPE: nucleotide

SEQUENCE LENGTH: 25 base pairs

STRANDEDNESS: single

TOPOLOGY: linear

MOLECULE TYPE: other nucleic acid (synthetic DNA for PCR)

TTGCCCCTGA AAATGGAAGT GGAGT 25

SEQ ID NO: 6

SEQUENCE TYPE: nucleotide

SEQUENCE LENGTH: 25 base pairs

STRANDEDNESS: single

TOPOLOGY: linear

MOLECULE TYPE: other nucleic acid (synthetic DNA for PCR)

AACAAATGCA ATAATTGATG CAAGC 25

SEQ ID NO: 7

SEQUENCE TYPE: nucleotide

SEQUENCE LENGTH: 25 base pairs

STRANDEDNESS: single

TOPOLOGY: linear

MOLECULE TYPE: other nucleic acid (synthetic DNA for PCR)

CTTTATGTTT TAGAGTCTCG GCTTG 25

SEQ ID NO: 8

SEQUENCE TYPE: nucleotide

SEQUENCE LENGTH: 25 base pairs

STRANDEDNESS: single

TOPOLOGY: linear

MOLECULE TYPE: other nucleic acid (synthetic DNA for PCR)

CGGAATTCCT TTATCAGCAA TTGTA 25

SEQ ID NO: 9

SEQUENCE TYPE: nucleotide

SEQUENCE LENGTH: 25 base pairs

STRANDEDNESS: single

TOPOLOGY: linear

MOLECULE TYPE: other nucleic acid (synthetic DNA for PCR)

TGTTGCCTAT GATCGACTAA TTACT 25

SEQ ID NO:10

SEQUENCE TYPE: nucleotide

SEQUENCE LENGTH: 25 base pairs

STRANDEDNESS: single

TOPOLOGY: linear

MOLECULE TYPE: other nucleic acid (synthetic DNA for PCR)

ACAATTACTG GACTTACTAA AATTG 25

SEQ ID NO:11

SEQUENCE TYPE: nucleotide

SEQUENCE LENGTH: 27 base pairs

STRANDEDNESS: single

TOPOLOGY: linear

MOLECULE TYPE: other nucleic acid (synthetic DNA for PCR)

TTAGGCATCA GGATTATCAA CATTAGC 27

SEQ ID NO:12

SEQUENCE TYPE: nucleotide

SEQUENCE LENGTH: 21 base pairs

STRANDEDNESS: single

TOPOLOGY: linear

MOLECULE TYPE: other nucleic acid (synthetic DNA for PCR)

GGGCACTAAT CCATCGAGGA T 21

SEQ ID NO:13

SEQUENCE TYPE: nucleotide

SEQUENCE LENGTH: 21 base pairs

STRANDEDNESS: single

TOPOLOGY: linear

MOLECULE TYPE: other nucleic acid (synthetic DNA for PCR)

GCTGAGTGAG CCAGTTATTT T 21

SEQ ID NO:14

SEQUENCE TYPE: nucleotide

SEQUENCE LENGTH: 21 base pairs

STRANDEDNESS: single

TOPOLOGY: linear

MOLECULE TYPE: other nucleic acid (synthetic DNA for PCR)

TTCCATAATT CCATCCTGGG A 21

SEQ ID NO:15

SEQUENCE TYPE: nucleotide

SEQUENCE LENGTH: 39 base pairs

STRANDEDNESS: single

TOPOLOGY: linear

MOLECULE TYPE: other nucleic acid (synthetic DNA for PCR)

GGAAGCCCGG GATGAAAAAA ATGCTTAGAA AAAAATTCT 39

SEQ ID NO:16

SEQUENCE TYPE: nucleotide

SEQUENCE LENGTH: 37 base pairs

STRANDEDNESS: single

TOPOLOGY: linear

MOLECULE TYPE: other nucleic acid (synthetic DNA for PCR)

GGAAGTCGAC TAACTTTTAG AAATTTTAGG CATCAGG 37

SEQ ID NO:17

SEQUENCE TYPE: nucleotide

SEQUENCE LENGTH: 37 base pairs

STRANDEDNESS: single

TOPOLOGY: linear

MOLECULE TYPE: other nucleic acid (synthetic DNA for PCR)

GGAACCCGGG TTGTATAATT GAATTAACTT GATTTGA 37

SEQ ID NO:18

SEQUENCE TYPE: nucleotide

SEQUENCE LENGTH: 37 base pairs

STRANDEDNESS: single

TOPOLOGY: linear

MOLECULE TYPE: other nucleic acid (synthetic DNA for PCR)

GGAACCCGGG TAACTTTTAG AAATTTTAGG CATCAGG 37

## Claims

1. A DNA encoding a surface antigen of Mycoplasma hyopneumoniae, which has an amino acid sequence represented by the following formula (I):

```
Met Lys Lys Met Leu Arg Lys Lys Phe Leu Tyr Ser Ser Ala Ile Tyr
                5                  10                  15
Ala Thr Ser Leu Ala Ser Ile Ile Ala Phe Val Ala Ala Gly Cys Gly
            20                  25                  30
Gln Thr Glu Ser Gly Ser Thr Ser Asp Ser Lys Pro Gln Ala Glu Thr
        35                  40                  45
Leu Lys His Lys Val Ser Asn Asp Ser Ile Arg Ile Ala Leu Thr Asp
    50                  55                  60
Pro Asp Asn Pro Arg Trp Ile Ser Ala Gln Lys Asp Ile Ile Ser Tyr
65                  70                  75                  80
Val Asp Glu Thr Glu Ala Ala Thr Ser Thr Ile Thr Lys Asn Gln Asp
                85                  90                  95
Ala Gln Asn Asn Trp Leu Thr Gln Gln Ala Asn Leu Ser Pro Ala Pro
            100                 105                 110
Lys Gly Phe Ile Ile Ala Pro Glu Asn Gly Ser Gly Val Gly Thr Ala
        115                 120                 125
Val Asn Thr Ile Ala Asp Lys Gly Ile Pro Ile Val Ala Tyr Asp Arg
    130                 135                 140
Leu Ile Thr Gly Ser Asp Lys Tyr Asp Trp Tyr Val Ser Phe Asp Asn
145                 150                 155                 160
```

Glu Lys Val Gly Glu Leu Gln Gly Leu Ser Leu Ala Ala Gly Leu Leu

165 170 175

Gly Lys Glu Asp Gly Ala Phe Asp Ser Ile Asp Gln Met Asn Glu Tyr

180 185 190

Leu Lys Ser His Met Pro Gln Glu Thr Ile Ser Phe Tyr Thr Ile Ala

195 200 205

Gly Ser Gln Asp Asp Asn Asn Ser Gln Tyr Phe Tyr Asn Gly Ala Met

210 215 220

Lys Val Leu Lys Glu Leu Met Lys Asn Ser Gln Asn Lys Ile Ile Asp

225 230 235 240

Leu Ser Pro Glu Gly Glu Asn Ala Val Tyr Val Pro Gly Trp Asn Tyr

245 250 255

Gly Thr Ala Gly Gln Arg Ile Gln Ser Phe Leu Thr Ile Asn Lys Asp

260 265 270

Pro Ala Gly Gly Asn Lys Ile Lys Ala Val Gly Ser Lys Pro Ala Ser

275 280 285

Ile Phe Lys Gly Phe Leu Ala Pro Asn Asp Gly Met Ala Glu Gln Ala

290 295 300

Ile Thr Lys Leu Lys Leu Glu Gly Phe Asp Thr Gln Lys Ile Phe Val

305 310 315 320

Thr Gly Gln Asp Tyr Asn Asp Lys Ala Lys Thr Phe Ile Lys Asp Gly

325 330 335

Asp Gln Asn Met Thr Ile Tyr Lys Pro Asp Lys Val Leu Gly Lys Val

340 345 350

Ala Val Glu Val Leu Arg Val Leu Ile Ala Lys Lys Asn Lys Ala Ser

355 360 365

Arg Ser Glu Val Glu Asn Glu Leu Lys Ala Lys Leu Pro Asn Ile Ser
370 375 380
Phe Lys Tyr Asp Asn Gln Thr Tyr Lys Val Gln Gly Lys Asn Ile Asn
385 390 395 400
Thr Ile Leu Val Ser Pro Val Ile Val Thr Lys Ala Asn Val Asp Asn
405 410 415
Pro Asp Ala.

**2.** The DNA of claim 1 wherein it comprises a DNA sequence represented by the following formula (II):

```
ATGAAAAAAA TGCTTAGAAA AAAATTCTTG TATTCATCAG CTATTTATGC AACTTCGCTT    60
GCATCAATTA TTGCATTTGT TGCAGCAGGT TGTGGACAGA CAGAATCAGG TTCGACTTCA   120
GATTCTAAAC CACAAGCCGA GACTCTAAAA CATAAAGTAA GTAATGATTC TATTCGAATA   180
GCACTAACCG ATCCGGATAA TCCTCGATGA ATTAGTGCCC AAAAAGATAT TATTTCTTAT   240
GTTGATGAAA CAGAGGCAGC AACTTCAACA ATTACAAAAA ACCAGGATGC ACAAAATAAC   300
TGACTCACTC AGCAAGCTAA TTTAAGTCCA GCGCCAAAAG GATTTATTAT TGCCCCTGAA   360
AATGGAAGTG GAGTTGGAAC TGCTGTTAAT ACAATTGCTG ATAAAGGAAT TCCGATTGTT   420
GCCTATGATC GACTAATTAC TGGATCTGAT AAATATGATT GGTATGTTTC TTTTGATAAT   480
GAAAAAGTTG GCGAATTACA AGGTCTTTCA CTTGCGGCGG GTCTATTAGG AAAAGAAGAT   540
GGTGCTTTTG ATTCAATTGA TCAAATGAAT GAATATCTAA AATCACATAT GCCCCAAGAG   600
ACAATTTCTT TTTATACAAT CGCGGGTTCC CAAGATGATA ATAATTCCCA ATATTTTTAT   660
AATGGTGCAA TGAAAGTACT TAAAGAATTA ATGAAAAATT CGCAAAATAA AATAATTGAT   720
TTATCTCCTG AAGGCGAAAA TGCTGTTTAT GTCCCAGGAT GAAATTATGG AACTGCCGGT   780
CAAAGAATCC AATCTTTTCT AACAATTAAC AAAGATCCAG CAGGTGGTAA TAAAATCAAA   840
```

GCTGTTGGTT CAAAACCAGC TTCTATTTTC AAAGGATTTC TTGCCCCAAA TGATGGAATG 900
GCCGAACAAG CAATCACCAA ATTAAAACTT GAAGGATTTG ATACCCAAAA AATCTTTGTA 960
ACTGGTCAAG ATTATAATGA TAAAGCCAAA ACTTTTATCA AAGACGGCGA TCAAAATATG 1020
ACAATTTATA AACCTGATAA AGTTTTAGGA AAAGTTGCTG TTGAAGTTCT TCGGGTTTTA 1080
ATTGCAAAGA AAAATAAAGC ATCTAGATCA GAAGTCGAAA ACGAACTAAA AGCAAAACTA 1140
CCAAATATTT CATTTAAATA TGATAATCAA ACATATAAAG TGCAAGGTAA AAATATTAAT 1200
ACAATTTTAG TAAGTCCAGT AATTGTTACA AAAGCTAATG TTGATAATCC TGATGCCTAA 1260.

**3.** A method for diagnosing Mycoplasmal pneumoniae of swine comprising detecting Mycoplasma hyopneumoniae by a DNA hybridization method using a DNA probe which comprises the DNA sequence represented by the formula (I) as defined in claim 1.

**4.** A method for diagnosing Mycoplasmal pneumoniae of swine comprising detecting Mycoplasma hyopneumoniae by a DNA hybridization method using a DNA probe which comprises the DNA sequence represented by the formula (II) as defined in claim 2.

**5.** A method for diagnosing Mycoplasmal pneumoniae of swine comprising detecting Mycoplasma hyopneumoniae by a DNA hybridization method using a DNA probe which comprises at least part of the sequence comprising 1st to 414th bases of the DNA sequence represented by the formula (II) as defined in claim 2.

**6.** A DNA fragment for primer comprising a member selected from the group consisting of DNA sequences which comprise at least part of the sequence comprising 1st to 414th bases of the DNA sequence represented by the formula (II) as defined in claim 2 and complementary DNA sequences thereof.

**7.** The DNA fragment of claim 6, wherein it comprises 10 to 30 nucleotides.

**8.** The DNA fragment of claim 7, wherein it is selected from the group consisting of:

① 5' ATGAAAAAAA TGCTTAGAAA AAAAT 3' (SEQ ID NO: 3)
② 5' GACAGACAGA ATCAGGTTCG ACTTC AG 3' (SEQ ID NO: 4)
③ 5' TTGCCCCTGA AAATGGAAGT GGAGT 3' (SEQ ID NO: 5)
④ 5' AACAAATGCA ATAATTGATG CAAGC 3' (SEQ ID NO: 6)
⑤ 5' CTTTATGTTT TAGAGTCTCG GCTTG 3' (SEQ ID NO: 7) and
⑥ 5' CGGAATTCCT TTATCAGCAA TTGTA 3' (SEQ ID NO: 8).

**9.** A DNA fragment for primer comprising a member selected from the group consisting of DNA sequences each of which is a part of the sequence comprising 415th to 1260th bases of the DNA sequence represented by the formula (II) as defined in claim 2 and complementary DNA sequences thereof.

**10.** The DNA fragment of claim 9, wherein it comprises 10 to 30 nucleotides.

**11.** The DNA fragment of claim 10, wherein it is selected from the group consisting of:

⑦ 5' TGTTGCCTAT GATCGACTAA TTACT 3' (SEQ ID NO: 9)

⑧ 5' ACAATTACTG GACTTACTAA AATTG 3' (SEQ ID NO: 10), and

⑨ 5' TTAGGCATCA GGATTATCAA CATTAGC 3' (SEQ ID NO: 11).

**12.** A method for detecting Mycoplasma hyopneumoniae, comprising the steps of: adding two kinds of primers selected from the DNA fragments for primer comprising a member selected from (i) the group consisting of DNA sequences which comprise at least part of the sequence comprising 1st to 414th bases of the DNA sequence represented by the formula (II) as defined in claim 2 and complementary DNA sequences thereof; and (ii) the group consisting of DNA sequences each of which is a part of the sequence comprising 415th to 1260th bases of the DNA sequence represented by the formula (II) as defined in claim 2 and complementary DNA sequences thereof, to a sample to be examined, amplifying a target DNA by a PCR method, and then detecting the amplified DNA fragment.

**13.** The method of claim 12, wherein the detection of the DNA fragment is carried out by a DNA hybridisation method using a DNA probe which comprises the whole or a part of the DNA sequence represented by the formula (II) as defined in claim 2.

**14.** The method of claim 13, wherein the two kinds of primers are selelcted from a combination of Group A and Group B:

Group A:

① 5' ATGAAAAAAA TGCTTAGAAA AAAAT 3' (SEQ ID NO: 3)

② 5' GACAGACAGA ATCAGGTTCG ACTTCAG 3' (SEQ ID NO: 4)

③ 5' TTGCCCCTGA AAATGGAAGT GGAGT 3' (SEQ ID NO: 5), and

⑦ 5' TGTTGCCTAT GATCGACTAA TTACT 3' (SEQ ID NO: 9)

Group B:

④ 5' AACAAATGCA ATAATTGATG CAAGC 3' (SEQ ID NO: 6)

⑤ 5' CTTTATGTTT TAGAGTCTCG GCTTG 3' (SEQ ID NO: 7)

⑥ 5' CGGAATTCCT TTATCAGCAA TTGTA 3' (SEQ ID NO: 8)

⑧ 5' ACAATTACTG GACTTACTAA AATTG 3' (SEQ ID NO: 10), and

⑨ 5' TTAGGCATCA GGATTATCAA CATTAGC 3' (SEQ ID NO: 11).

**15.** A recombinant antigenic peptide which comprises the amino acid sequence represented by the formula (I) as defined in claim 1 or a part thereof.

**16.** A method for diagnosing Mycoplasmal pneumoniae of swine, wherein a peptide is brought into contact with a sample under the conditions that the peptide reacts with anti-M.hp antibodies present in the sample to form an immunological complex and the immunological complex is measured to confirm the existence of the antibodies in the sample, and wherein the peptide is a recombinant antigenic peptide which comprises the amino acid sequence represented by the formula (I) as defined in claim 1 or a part thereof.

**17.** The method of claim 16, wherein it is conducted by an ELISA method.

**18.** The method of claim 17, wherein it is conducted by a double sandwich method wherein an anti-M.hp monoclonal antibody is used in combination with the recombinant antigenic peptide.

# FIG. 1

Detection of M.hp by DNA Probe Method

(1) Undiluted Solution
(2) Diluted Soln. (1/10)
(3) Diluted Soln. (1/100)
(4) Diluted Soln. (1/1000)

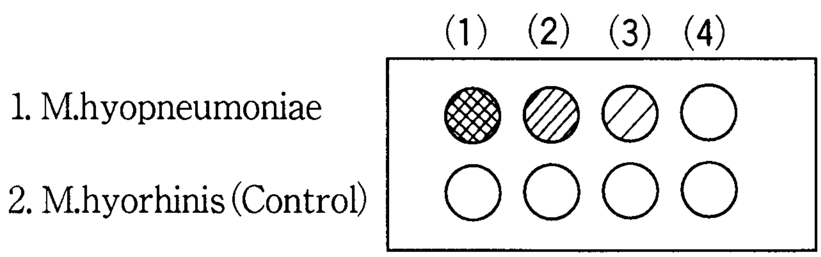

# FIG. 2

Detection of M.hp by DNA Probe Method

(1) Undiluted Solution
(2) Diluted Soln. (1／10)
(3) Diluted Soln. (1／100)
(4) Diluted Soln. (1／1000)

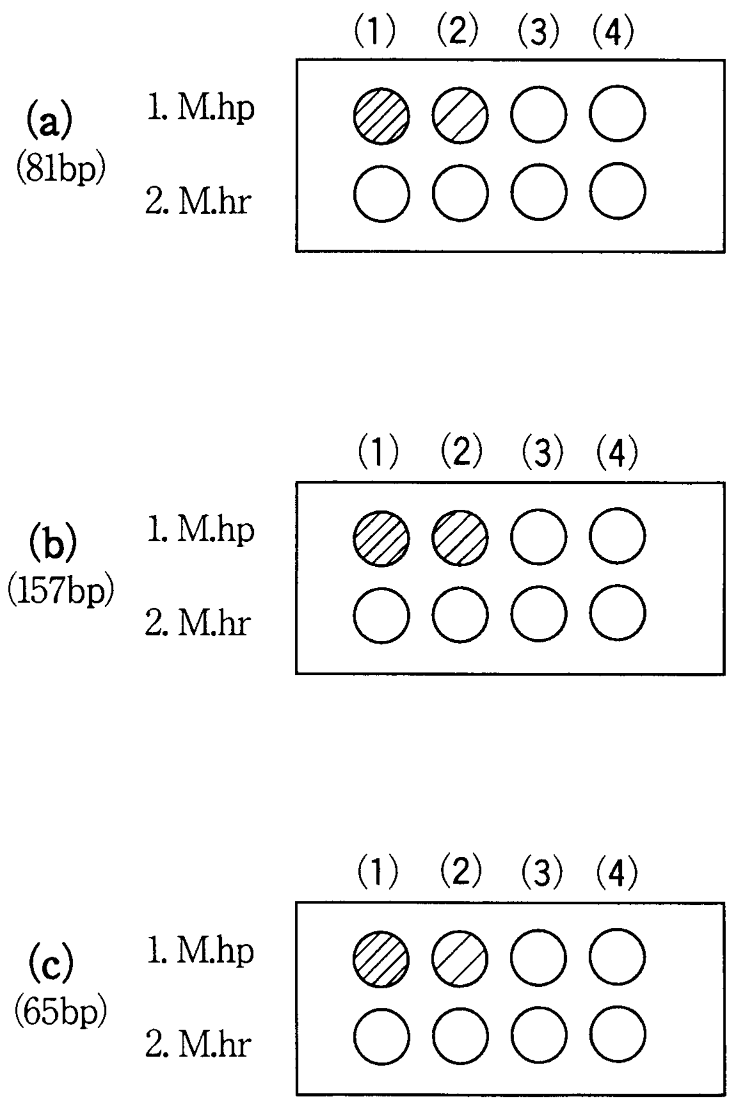

# FIG. 3

Detection of M.hp by PCR Method

Samples 1 and 6: Molecular Weight Marker
2: Plasmid 5ng
3: Plasmid 10ng
4: M.hp DNA10ng
5: M.hp DNA50ng
7: M.hp Bacterial Cell Lysate
8: M.hr Bacterial Cell Lysate
9: M.f Bacterial Cell Lysate

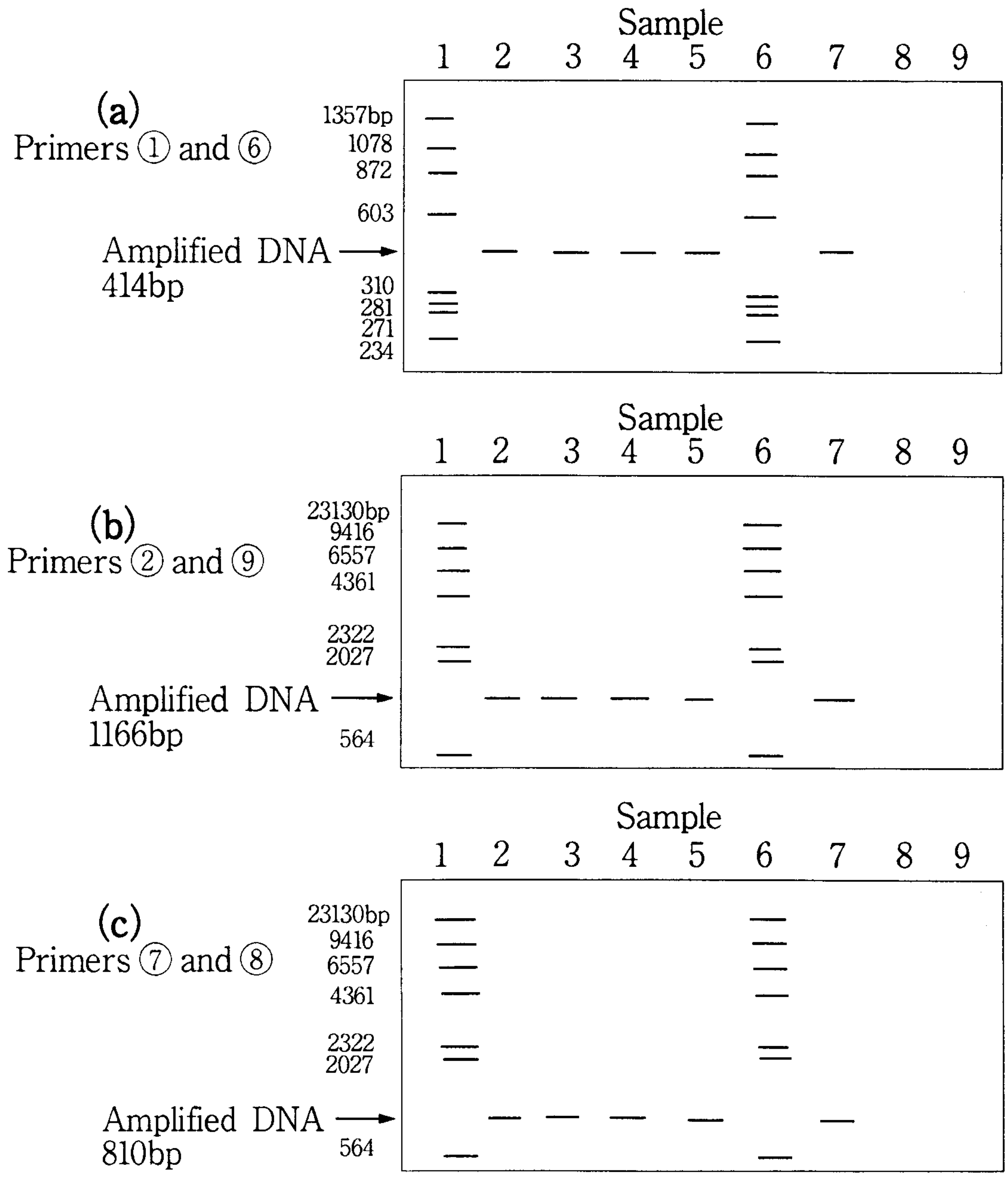

# FIG. 4

Detection of M.hp by PCR Method

1: Molecular Weight Marker
2: Tracheo Bronchial Lavage
3: Lung Homogenate

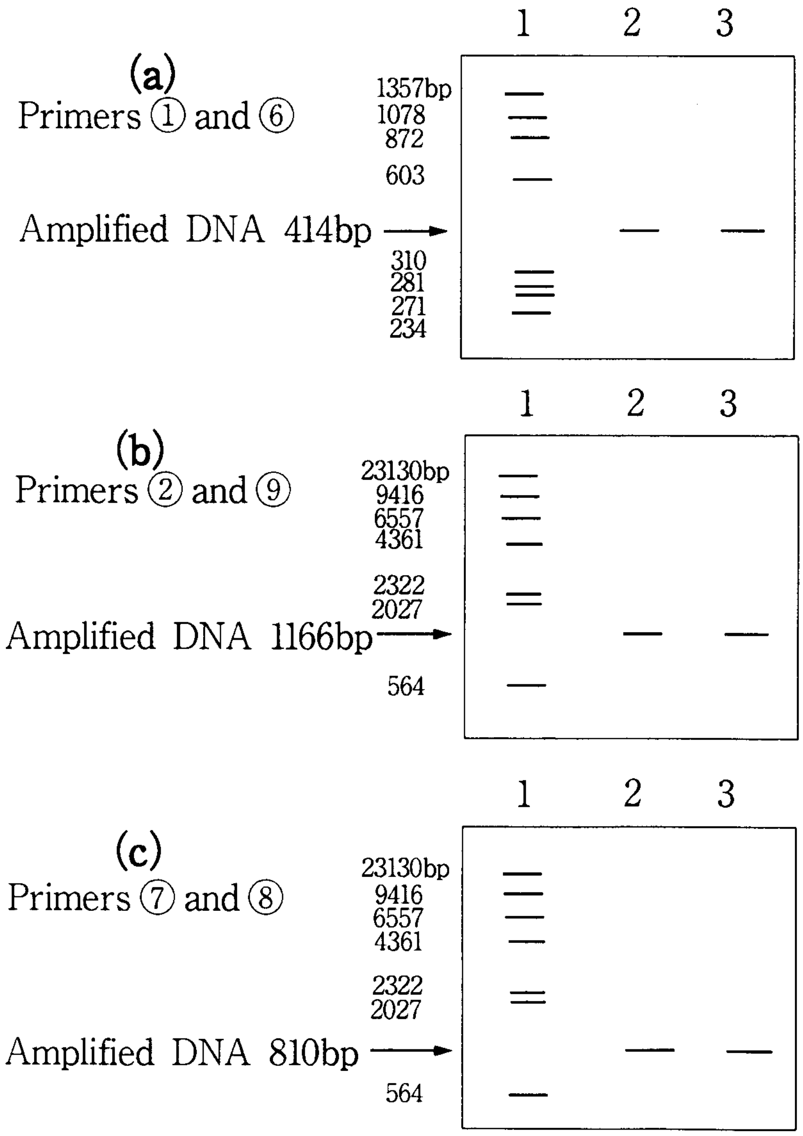

## FIG. 5

Detection of M.hp by PCR－DNA Probe Method

1: Tracheo Bronchial Lavage
2: M.hp Bacterial Cell Lysate
3: M.hr Bacterial Cell Lysate
4: M.f Bacterial Cell Lysate

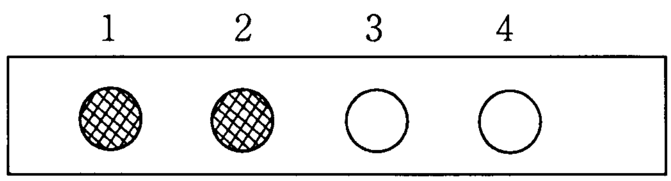

European Patent Office

# EUROPEAN SEARCH REPORT

Application Number

EP 91114335.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | JP - A - 2-167 079 (NIPPON FLOUR MILLS CO. LTD.) * Totality * | 1-18 | C 12 N 15/31<br>C 12 P 21/02<br>C 12 Q 1/68<br>G 01 N 33/541<br>C 07 K 13/00 |
| P,X | PATENT ABSTRACTS OF JAPAN, unexamined applications, C field, vol. 15, no. 211, May 29, 1991 THE PATENT OFFICE JAPANES GOVERNMENT page 133 C 836 * Kokai-no. 3-61 487 (NIPPON FLOUR MILLS CO LTD.) * | 1-18 | |
| P,Y | CHEMICAL ABSTRACTS, vol. 115, no. 5, August 5, 1991, Columbus, Ohio, USA STIPKOVITS et al.: "Recombinant plasmid for identification of Mycoplasma hyopneumoniae strains and for detection of Hyopneumoniae infection" page 250, left column, abstract-no. 43 462n & Hung. Teljes HU 54,205, 28 Jan 1991, Appl. 90/3,297, 01 Jun 1990 | 1-5 | TECHNICAL FIELDS SEARCHED (Int. Cl.5)<br>C 12 N<br>C 12 P<br>C 12 Q<br>G 01 N<br>C 07 K |
| Y | WO - A - 88/00 977 (SYNERGEN INC.) * Abstract; claim 1 * | 15-17 | |
| X | EP - A - 0 359 919 (ML TECHNOLOGY VENTURES L.P.) * Claims 1-8; abstract * | 15-17 | |
| X | EP - A - 0 196 215 (BIOGEN N.V.) * Claims 1-3,10,12,17,18 * | 15-17 | |

The present search report has been drawn up for all claims

| Place of search | Date of completion of the search | Examiner |
|---|---|---|
| VIENNA | 25-11-1991 | AUGUSTIN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document